# EUROPEAN PATENT SPECIFICATION

(11) **EP 1 861 711 B1**
(45) Date of publication and mention of the grant of the patent: **11.01.2012**
(21) Application number: 06723224.9
(22) Date of filing: 06.03.2006
(51) Int. Cl.: G01N 33/566

(54) **USE OF MGC4504**
VERWENDUNG VON MGC4504
UTILISATION DE MGC4504

(30) Priority: 11.03.2005 EP 05005383
(43) Date of publication of application: 05.12.2007
(73) Proprietor: SANOFI, 75013 Paris (FR)
(72) Inventor: VOSS, Marc, Dietrich, D-65926 Frankfurt am Main (DE); TSCHANK, Georg, 55270 Essenheim (DE); KORN, Marcus, Hermann, 63065 Offenbach (DE)
(74) Representative: Schneider, Rudolf
(86) International application number: PCT/EP2006/002020
(87) International publication number: WO 2006/094735

(56) References cited:
- EP-A1- 1 010 432
- WO-A-00/26245
- WO-A-2005/072076
- WO-A2-02/098441
- US-A1- 2003 224 988
- DATABASE ENTREZ NUCLEOTIDE, [Online] NCBI; nucleic acids number 55-74 2 January 1998 (1998-01-02), MARRA, M, ET. AL.: "The WashU-HHMI Mouse EST Project" XP002388326 Database accession no. AA727648
- SRINIVASAN JAGAN ET AL: "AppaDB: An AcedB database for the nematode satellite organism Pristionchus pacificus." NUCLEIC ACIDS RESEARCH, vol. 32, no. Database Issue, 1 January 2004 (2004-01-01), pages D421-D422, XP002388324 ISSN: 0305-1048 & DATABASE ENTREZ NUCLEOTIDE, [Online] NCBI; nucleic acids 83-97 9 July 2004 (2004-07-09), SRINIVASAN, J., OTTO, G.W., KAHLOW, U., GEISLER, R. AND SOMMER, R.J.: Database accession no. CL655602
- CROSS SALLY H ET AL: "Purification of CpG islands using a methylated DNA binding column" NATURE GENETICS, vol. 6, no. 3, 1994, pages 236-244, XP000578157 ISSN: 1061-4036 & DATABASE ENTREZ NUCLEOTIDE, [Online] NCBI; nucleic acids 146-160 18 October 1995 (1995-10-18), MACDONALD,M., HUCKLE,E., WILKINSON, P., AND MICKLEM,G.: "Direct submission" Database accession no. Z58073

### SEQUENCE LISTING

<110> sanofi-Aventis Deutschland GmbH
<120> Use of MGC4504
<130> DE 2005/008
<140> 05005383.4 <141> 2005-03-11
<160> 14
<170> PatentIn Ver. 2.1
<210> 1
   <211> 1528
   <212> DNA
   <213> Homo sapiens
<400> 1
   ggcacgaggc aggccaggtg tgtgcgtccg tcggtctttc cgtgcccacg ccggagacca 60
   gccccggagg ccgcctgggc ctatccctgt gccaggcacc atgaagcagg agtctgcagc 120
   cccgaacacc ccgcccacct cgcagtcccc tacgccgtcc gctcagttcc cccgaaacga 180
   cggcgaccct caagcgctgt ggattttcgg gtacggctcc ctggtgtgga ggcccgactt 240
   cgcctacagc gacagccgtg tgggcttcgt gcgcggctac agccgccgtt tctggcaggg 300
   agacaccttc catcggggca gcgacaagat gcctggccgt gtggtgacgc tccttgaaga 360
   tcatgagggc tgcacttggg gcgtggcata ccaagtgcaa ggggagcagg taagcaaggc 420
   cctgaagtac ctgaatgtgc gagaggcagt gcttggtggc tacgatacca aggaggtcac 480
   cttctatccc caagatgctc ctgaccaacc actgaaggca ttggcctatg tggccacccc 540
   acagaaccct ggttacctgg gccctgcgcc tgaagaggcc attgccacgc agatcctggc 600
   ctgccggggc ttctccggcc acaaccttga atacttgctg cgtctggcag acttcatgca 660
   gctctgtggg cctcaggcgc aggacgagca cctggcagcc atcgtggacg ctgtgggcac 720
   catgttgccc tgcttctgcc ccaccgagca ggctctggcg ctggtgtgag gggctgagcc 780
   cctgcgggga gtgctcatgt ggacatcagg gccagacacc cactccagtg cacaagacag 840
   acttgcgacc gcttgagccc actgagcaga tatggtgggt ggctggaggc ttctctttct 900
   cagtccctgc ctgtctgcca gcctgcagct ctcctgcttg acactgactt actacttgaa 960
   actttattta ttgcaccatg ttggtgtggt gggcaggtgg agggcctgcc ctggacacag 1020
   gggccctgct gagcagtggc cccatcctgg aacttgacca gattcccccc agtgctgctg 1080
   ctaaccccac accacccagg cctccacctc cccagggagt ctccaagagc ctcgatcctc 1140
   tgctcactca gcccagccat ccatagccct gggaattcca cctgccaagg atcccagcag 1200
   gctggatgag ggatagtagg gcatgaggag aaggagccct gtaaggactg aggccccggc 1260
   cagcccttct cctccaccag ttccccagag cagagctgga gctgatgcct ggacacagct 1320
   gctgagcctg gcctgggcct cttacccact tggttgtttt cttgtccctc tgtctgtctg 1380
   tctatctact tgtctgtctg ggccactcct gcctgtgtgt tggtctattc ctgggaagct 1440
   catcactaca ggccctggca accttcccag tctgtcccat actgttaccc ataaaactat 1500
   ctctttaaaa aaaaaaaaaa aaaaaaaa                                   1528
<210> 2
   <211> 222
   <212> PRT
   <213> Homo sapiens
<400> 2
   Met Lys Gln Glu Ser Ala Ala Pro Asn Thr Pro Pro Thr Ser Gln Ser
   1               5                   10                  15
   Pro Thr Pro Ser Ala Gln Phe Pro Arg Asn Asp Gly Asp Pro Gln Ala
               20                  25                  30
   Leu Trp Ile Phe Gly Tyr Gly Ser Leu Val Trp Arg Pro Asp Phe Ala
           35                  40                  45
   Tyr Ser Asp Ser Arg Val Gly Phe Val Arg Gly Tyr Ser Arg Arg Phe
       50                  55                  60
   Trp Gln Gly Asp Thr Phe His Arg Gly Ser Asp Lys Met Pro Gly Arg
   65                  70                  75                  80
   Val Val Thr Leu Leu Glu Asp His Glu Gly Cys Thr Trp Gly Val Ala
                   85                  90                  95
   Tyr Gln Val Gln Gly Glu Gln Val Ser Lys Ala Leu Lys Tyr Leu Asn
               100                 105                 110
   Val Arg Glu Ala Val Leu Gly Gly Tyr Asp Thr Lys Glu Val Thr Phe
           115                 120                 125
   Tyr Pro Gln Asp Ala Pro Asp Gln Pro Leu Lys Ala Leu Ala Tyr Val
       130                 135                 140
   Ala Thr Pro Gln Asn Pro Gly Tyr Leu Gly Pro Ala Pro Glu Glu Ala
   145                 150                 155                 160
   Ile Ala Thr Gln Ile Leu Ala Cys Arg Gly Phe Ser Gly His Asn Leu
                   165                 170                 175
   Glu Tyr Leu Leu Arg Leu Ala Asp Phe Met Gln Leu Cys Gly Pro Gln
               180                 185                 190
   Ala Gln Asp Glu His Leu Ala Ala Ile Val Asp Ala Val Gly Thr Met
           195                 200                 205
   Leu Pro Cys Phe Cys Pro Thr Glu Gln Ala Leu Ala Leu Val
       210                 215                 220
<210> 3
   <211> 4324
   <212> DNA
   <213> Homo sapiens
<400> 3
   tgcacatcat gtgcgactct gcaaggtgga ggtgatgcct gggcccatcg ctgctcataa 60
   actggcacct ctgtgggtat gacatcggcg gccccttcct gtggggcgtt agaaggaaag 120
   acgggaaggg tttgccgttg aagttacaga gaggaaccag ctaagtgagt ggtttgctgc 180
   ctgggagtgt cctgctggga ggaaaagaga cagatgttgt tggcaagaat aagcaaggaa 240
   tgggaacagg aaagacatgt tcccagggag acattgcaga tcccctgggc cagcttctgt 300
   ggagattcct attttgaggg tcaaggttga acttgtgtta cacaggacat gggatatagc 360
   cctggccttt tccaagcaat agcctcactt attctaccta tatttgtgga ggatcttcca 420
   catgcctggc ttgtgttggt tctcttgggc cttcaaccat gaaccatact ctggggaagt 480
   gctcaggtga gtgagaggca ggacgcaaac gattcccagc aaggaggctc tggagctggc 540
   ccagctgcct tcagatccag gctccactac tttctgccac gtcaccaaca gggtttacct 600
   agctttcctg catgcttatc tgtgaaatga tatttacctc acaaagtagt gaagaaatgt 660
   gtaattagaa tggatgatgt aaaggcgttt agcaaattgc ctggcacatg ttatacacat 720
   aataaatgag tgtttctgtt gtaagcacga gttggaaggg acagataagt aaactgcagt 780
   ggcgtaaagt gttgtgggag tgtagaggag ggagtgtctg actctgccag gagcaagatc 840
   aggttattga ggacattgcc aaaagagtta tgaaacactg ggactttggt cagggtggga 900
   ggaggtctga gaacgggcat catgaaaatg ttccctaaat gtttcaggta tgctatccat 960
   ccctccccct ccagaaaacc actaatgact ctttaaaagt aacttttttt ttttttttta 1020
   gacaggatct tgctctgttg cccaggttag agagcagtgg cacaatcatg gctcactaca 1080
   gcctcgacct cctgggctca agcaaccctc ctgcctcggt ctcctgagta gctgggacta 1140
   caggtgcatg ttgctaagcc cggctaattt ttaaaaattt ttttgtagag atgaggtttc 1200
   attatgtttc ccaggctggt ctcaaactcc tgagctcaag tgatcctcct gcttggcctc 1260
   ccaaagtgct gagattacag gtgtgaacca ccacgcccag caaaaattaa cttattatta 1320
   tttaaaaatt acttttcata aaacttggaa aatagaggaa agaattgctc acaatcccaa 1380
   cacactagta tttgctgcct ccctatcttt attgttactg tttttaagtt ttaatcatag 1440
   ttcatgtgaa aatttatatt ttgcattttt gccttaatat gcaatcatac acatgtttac 1500
   tacgtttttt ttgtttgttt tgtttgtttg tttcccgaga caaactcgtg ctctgttgcc 1560
   caggctggac tgcagtggtg ggatcttggc tcactgcaac ctctgcctcc tgggttgaag 1620
   taattctcct gcctcagcct cccgagtaac tgggatttca ggcacctgcc accatgcccg 1680
   gctaattttt tttgtatttt tagaagagac ggggtttcac catgttgccc aggctggtct 1740
   cgaactcctg agctcaggca gtctgcccac cttggcctcc catagtgcta agactactgg 1800
   cgtgagccac tgcacccggc ccaacataca tattgtttct aatgtctgca caaatttaat 1860
   ggactatatc atatttatgt aactaattat ctattgctgg gtatttaggt tgttttcttt 1920
   gtgtttgtgt gtgtgtgtat gtgcacattt ttctgtcatg tgaatggtaa attgacataa 1980
   tttccttaaa catttagcta attttggata tttaagtagt ttttcttctt ttgtttttgc 2040
   tataatatgt aaaggtcggg cgtggtggct cacgcctgta atctcagcac tttgggaggc 2100
   caaggtgggc ggatcacaaa atcaggagtt tgagatcagc ctggccaata tggcgaaacc 2160
   tcgtgtctac taaaaataca aaaattatcc aggcgtggtg gcaggtgcct gtagtcccag 2220
   ctactcggga ggctcaggca ggagaatcgc ttgaacctgg gaggcggagg ttgcagtgag 2280
   ccgagattgt gccactgcac tccagcctgg gcaacagaga gagactccac ctcaaaaaaa 2340
   aaaaaaaaaa aaaaaaaaaa aaaaaaaagt aacaatgcat tgaaaatctt caggcatatg 2400
   gcttttcccg tattttgaat aatttcctga aggagtattg ggtcaaaggg cagggatatt 2460
   ttgtgattct tgacttgtat caccaagctg ctttctcaaa gggttgtttg tgccaattca 2520
   gatacttcag caatatgtgg ataccagttt cccacttccc ggactacaac ccacagctgg 2580
   tgagtggaaa agaggacatt cttctaagaa gctaccaaca aaggaggtgc tcatatgaac 2640
   gaaaggggac aggatagaag agaagaggga gactgaatgt gggaaatgga gagaaagcga 2700
   aaaagaaagg aataaccaga aaggaccatt tgctggtaat tttttttttt tcctgattgg 2760
   aaaagtaatt catgctccct gtaaaaatcc agactttttc attactgttt aagtttgata 2820
   ctgaaagtcc tcctctggtg ccagcctctg ctaactgttt aggtatattc ttccagatta 2880
   tttattacac gaatactttt tgtttttaaa gaaatagctt tgtactatac acactgttgt 2940
   gcaagtttcc cacttatatc tgcgacacct tcggaatgaa tatgtgaagt gtgaagatca 3000
   cgtttagttt aaatattgaa ttagaatcga atgagaagcc atgttgcaca ttgcccctgc 3060
   cagccatctc ctggccagtt tagagagaag gccttcagct gcctctgttt cccttttaca 3120
   gtcagtgaca tcccctgagg gcagtccctt gctgcgtatt gggtcaaccc ccggcggcct 3180
   gtggactcgg ctgggaggcc agaagccgag ggagaagacc agtagctggc catagtcttg 3240
   tgggggcctc actgggccct ccttgcccgc tgctcccgga accttgtgcc tatggattaa 3300
   tcattgctgc aaaatctcac gtccaggaag aattaaaccc atcgccttgg gggcaagact 3360
   agcctggctt ttgcatcttt atagcctttc tccgcaggtg cagccaccac ctccaaagtg 3420
   actgccggtc ggtccaagcg ctctaacagg ctcttccccg agcctacctg ggctcgaggg 3480
   gtcccacacc tggtccgggg cgtagcccat gtgaggcctg caagctccgg gcgcctggcc 3540
   tacctaactc agccccgcag tctctgcaga accccctgca gccccgtggc ccagtttctc 3600
   catttgctca caatggccaa cggcgtgagg cacttgagcg gaatgcggac ggtgcctccg 3660
   acagctttcc cgcagtgcat gatctcagga ggggccccag agacggaagt ttacgacgcc 3720
   cagagcgctc cttctggacg tgccctggac tcgacttggg actgagtctg tgccgcctag 3780
   gctggcaggg actcggccag atgcactagc ggcaaggctg ggctgacacc gcctgggact 3840
   cgctcgccgt acctccaagg ggcccaggtc atgagggggc ggctggggag acgcccgccc 3900
   ggtgcccgcc ctgggcccgg cgggtgcccc ctgcccgccg cctgacgcaa tccgaggcgc 3960
   ccttgctgca tgccggcgct ggagaaacct cccctgccgt tgcatcactc ctgccaggtt 4020
   tgccacagag gtggagctga accaatcagc gggcgcgctg ggcccgcggc gcggttaagt 4080
   gagaagcaga atccgaagtt gattggccaa aaggggattt gccccacccc aatcaccgcg 4140
   gcagctggcc gcggtgggcg tggtctcccg gcaagaaggt ttaaagggcg cctccatggg 4200
   gggcgctcag ctggagctac cgagcggtgc caggccaggt gtgtgcgtcc gtcggtcttt 4260
   ccgtgcccac gccggagacc agccccggag gccgcctggg cctatccctg tgccaggcac 4320
   catg                                                              4324
<210> 4
   <211> 24
   <212> DNA
   <213> Artificial sequence
<220>
   <223> Description of Artificial sequence: primer
<400> 4
   acttcgcctc cttctctgct ctcc 24
<210> 5
   <211> 23
   <212> DNA
   <213> Artificial Sequence
<220>
   <223> Description of Artificial sequence:primer
<400> 5
   ggccctgcta atcccacact acc 23
<210> 6
   <211> 22
   <212> DNA
   <213> Artificial sequence
<220>
   <223> Description of Artificial sequence:primer
<400> 6
   aagtccctca ccctcccaaa ag 22
<210> 7
   <211> 22
   <212> DNA
   <213> Artificial Sequence
<220>
   <223> Description of Artificial sequence:primer
<400> 7
   cctcaacacc tcaaaccact cc 22
<210> 8
   <211> 20
   <212> DNA
   <213> Artificial Sequence
<220>
   <223> Description of Artificial Sequence:primer
<400> 8
   ggcagggaga caccttccat 20
<210> 9
   <212> DNA
   <213> Artificial Sequence
<220>
   <223> Description of Artificial Sequence:primer
<400> 9
   tgcagccctc atgatcttca 20
<210> 10
   <211> 15
   <212> DNA
   <213> Artificial sequence
<220>
   <223> Description of Artificial Sequence:primer
<400> 10
   gctgccccga tggaa 15
<210> 11
   <211> 32
   <212> DNA
   <213> Artificial sequence
<220>
   <223> Description of Artificial Sequence:primer
<400> 11
   cgggatcccg catgaagcag gagtctgcag cc 32
<210> 12
   <211> 62
   <212> DNA
   <213> Artificial Sequence
<220>
   <223> Description of Artificial Sequence:primer
<400> 12
   cgggatcccg catgtaccca tacgacgtcc cagactacgc tatgaagcag gagtctgcag 60
   cc                                                                62
<210> 13
   <211> 33
   <212> DNA
   <213> Artificial Sequence
<220>
   <223> Description of Artificial Sequence:primer
<400> 13
   ccggaattcc ggtcacacca gcgccagagc ctg 33
<210> 14
   <212> DNA
   <213> Homo sapiens
<400> 14

## Description

The present invention concerns the use of MGC4504 for the identification of pharmaceutical substances. Further aspects of present invention concern methods for the identification of said substances and the use of MGC4504 for the preparation of a medicament. The present invention, thereby, concerns the prevention or treatment of the malfunction of the carbohydrate/glucose or lipid metabolism.

Diabetes mellitus is a frequent malfunction of the endocrine system. The term diabetes comprises different forms of malfunctions of the glucose and lipid metabolism having in common a relative or absolute lack of insulin, as well as impaired insulin action. Type 1 diabetes mellitus is an autoimmune-mediated destruction of pancreatic beta-cells resulting in insulin deficiency and hyperglycemia. The most common form, type 2 diabetes mellitus, Is characterized by dysregulations In lipid and glucose metabolism, associated with obesity, insulin resistance and the metabolic syndrome. Initially, the development of type 2 diabetes is associated with insulin resistance, but normoglycemia and normal pancreatic insulin secretion. Disease progression is associated with hyperglycemia and loss of pancreatic beta cell mass, finally also resulting in insulin deficiency and hyperglycemia. With more than 6% of the US population suffering from diabetes (i.e. more than 18 million people in 2004) and future predictions of increasing numbers worldwide, an ever increasing population of diabetes patients has to be faced, whose quality of life is severely affected. In addition, diabetes carries a tremendous socio-economic burden with high direct and indirect costs.
WO 00/26245 discloses human membrane transport proteins (MTPR) and polynucleotides coding therefor and the use thereof for the treatment of disorders associated with decreased expression or activity of MTPR and for diagnostic purposes. One of the disclosed polynucleotides and proteins is identical to MGC4504.
WO 02/0984401 discloses methods for preventing or treating metabolic disorders such as metabolic syndrome X, body weight disorder or non-insulin dependent diabetes and discloses in particular manmalian neuronatin proteins or fragments thereof for use for preventing or treating such metabolic disorders.

Therefore, there is a great need of novel pharmaceutical substances for the prevention and/or the treatment of diseases associated with or caused by diabetes.

The malfunction of the carbohydrate/glucose or lipid metabolism refers, throughout the specification, to metabolic syndrome, obesity, dislipidemia, diabetes mellitus type I or II or insulin resistance and preferably insulin resistance.

Thus, it is the object of present invention to provide novel means of identifying substances active in the prevention and/or treatment of diabetes.

This is achieved by the use of MGC4504 protein, a functional derivative or fragment thereof, or a nucleic acid coding for said protein, derivative or fragment, for the identification of substances active in preventing or treating a disease associated with or caused by a malfunction of the glucose or lipid metabolism.

The present invention is based on results of the inventors, for the first time providing evidence of the implication of MGC4504 (Q9BUX1) in the pathological processes underlying diabetes, especially type 2 diabetes mellitus. So far, there has been no knowledge of a potential implication of MGC4504 of disease states associated with or caused by an impaired carbohydrate or lipid metabolism. By showing that MGC4504 expression levels are decreased in an animal model of insulin resistance and type 2 diabetes and providing experimental evidence that an increased expression of MGC4504 leads to increased cellular insulin sensitivity, the inventors demonstrated for the first time the functional implication of MGC4504 In this physiological context. Thus, substances able to modulate MGC4504 activity must be considered to have a fundamental impact on the pathologic state and progress of diseases associated or caused by malfunctions of these mechanisms.

In its broadest sense the present inventions pertains to methods and tools as defined in the appended claims.

MGC4504 (entry QSBUX1 in the trEMBL data base) is a protein of 222 amino acids containing a putative Chac-ilke domain (aa 32-208), but no transmembrane domains, according to standard sequence predictions. MGC4504 amino acid sequence is highly conserved between humans, rat and mouse (> 85% amino acid Identity). So far, no molecular function has been assigned to MGC4504 in literature.

The gene locus of MGC4504 is on chromosome 15. The genomic sequence of MGC4504 is publicly available at the NCBI nucleotide-database under: AC_020661 (SEQ ID No.3). The coding polynucleotide sequence of the MGC4504 is available under the following entry number at the NCBI nucleotide-database: NM_024111 (sequence ID No.1). The derived protein sequence is available under the same following entry number at the NCBI nucleotide-database: NM_024111 (sequence ID No.2). NCBI is the National Centre for Biotechnology Information (postal address: National Centre for Biotechnology Information, National Library of Medicine, Building 38A, Bethesda, MD 20894, USA; internet address: www.ncbi.nhm.nih.gov).

The use according to present invention allows for the identification of novel substances for the prevention and/or treatment of diseases associated with an impaired glucose or lipid metabolism. The use according to present invention comprises the identification of substances with the desired characteristics as well as the further characterization of substances already identified to be useful for the prevention and/or treatment of diseases associated with an impaired glucose or lipid metabolism (i.e. the use according to present invention is useful for e.g. compound screening as well as compound profiling).

A substance as to be employed for the different aspects of present invention can be any biological or chemical substance or natural product extract, either purified, partially purified, synthesized or manufactured by means of biochemical or molecular biological methods.

A substance considered as being active in preventing or treating a disease associated with or caused by a malfunction of the carbohydrate or lipid metabolism in the sense of the different aspects of present invention can be any substance having an influence of one of the functions of MGC4504 or on the MGC4504 amount or steady state level in a cell or on MGC4504 expression.

To this end, the substance can modulate any of the functions of MGC4504 (e.g. those as defined below). MGC4504 protein activity can be modulated by the substance e.g by direct interaction and interference of MGC4504 polypeptide/protein or fragments thereof. The substance can also modulate MGC4504 expression, e.g. on the level of transcription (initiation, elongation, processing, etc), transcript stability, translation. Moreover it can modulate the posttranslational modification, protein folding etc. of MGC4504. The substance can exert the above effects directly or indirectly (indirectly meaning i.e. by interfering (positively or negatively) with natural signalling cascades having influence on MGC4504 function / protein activity / expression etc.). Moreover the substance can also mimick MGC4504 activity (i.e. take over its function/role).

A fragment of MGC4504 can be any polypeptide or polynucleotide that is shorter than the corresponding wild type, e.g. shorter than homo sapiens (hs) MGC4504 according to the polypeptide of SEQ ID No.2 or one of the polynucleotides according to SEQ ID No. 1 or SEQ ID No. 3 or No. 14.

A derivative of MGC4504 or of a MGC4504 fragment can be any modification of an MGC4504 polynucleotide, polypeptide or of a fragment thereof. Derivatives comprise modifications of the amino acid or nucleotide sequence leading to the stabilization of the polynucleotide, being phosphoorothioate modifications or facilitating its entry into or uptake by cells by coupling the protein or fragment thereof to cell-permeant phosphopeptides, such as ortho coupling to cell-permeant peptide vectors, e.g. based on the antennapedia/penetratin, TAT, and signal-peptide based sequences.

A functional fragment of MGC4504 is any fragment (either polypeptide or polynucleotide), which exhibits at least one of the functions of MGC4504, as indicated above.

The term "functional derivative" of MGC4504 comprises any kind of modification of MGC4504 with respect to the naturally occurring form (either polypeptide or polynucleotlde), which at least has one of the functions of MGC4504. The present invention also comprises functional derivatives of fragments of MGC4504.

Functions of MGC4504 comprise the capability of improving / increasing insulin release from pancreatic beta cells, improving/increasing insulin-stimulated glucose disposal (e.g. glucose uptake; glycogen synthesis and insulin sensitivity) influencing , insulin signalling or insulin action (e.g. Akt phosphorylation, GLUT4 translocation, aPKC activiation; activation of down-stream signaling cascades leading to improved glucose uptake). Functions of MGC4504 preferably concern the capability of increasing the insulin-stimulated glucose uptake.

The term "activity of MGC4504" relates the protein activity of MGC4504 protein, polypeptide or fragments thereof (e.g. one of those as involved in the functions defined above).

A substance capable of modulating MGC4504 activity is a substance considered as active in preventing or treating a disease associated with or caused by a malfunction of the carbohydrate or lipid metabolism in the context of present invention.

Another aspect of present invention concerns a method for identifying substances active in preventing or treating a disease associated with or caused by a malfunction of the carbohydrate or lipid metabolism comprising:
a. Contacting a cell, which has a decreased amount or activity of MGC4504 with a test substance;
b. Determining whether the test substance is able to increase the amount or activity of MGC4504 present in the cell.

Wherein a substance able to detectably increase the MGC4504 amount or activity Is considered a substance active in preventing or treating a disease associated with or caused by a malfunction of the carbohydrate or lipid metabolism.

The method can be a biochemical assay using isolated MGC4504 or extracts comprising MGC4504 or it can be a cellular assay.

A test substance can be any of the substances as defined above.

According to one preferred embodiment, the substance is capable of fully restoring the amount or activity of MGC4504.

A cell with a decreased amount or activity of MGC4504 can be any cell with a detectably lower amount or activity of MGC4504 (i.e. due to expression of a mutant MGC4504 protein with lowered function, or low level expression, etc.) in comparison to a reference cell, e.g. a cell with full MGC4504 activity (such as, for example HEK293). It can also be a cell totally devoid of MGC4504 or MGC4504 activity (e.g. due to expression of a loss of function MGC4504 mutant protein or due to a complete lack of MGC4504 expression). These comprise cells naturally having these characteristics as well as cells genetically modified to exhibit these characteristics, such as, e.g. genomic or inducible MGC4504 knock out cells, as well as cells expressing a dominant negative mutant of MGC4504, etc.

One example is a method for identifying substances active in preventing or treating a disease associated with or caused by a malfunction of the carbohydrate or lipid metabolism comprising:
a. Contacting a nucleic acid coding for a MGC4504 protein, derivative or fragment thereof with a test substance in a transcriptionally active system;
b. Determining the amount of mRNA coding for MGC4504 protein, derivative or fragment thereof present in said system in presence of said substance;
c. Determining the amount of mRNA coding for MGC4504 protein, derivative or fragment thereof present in said system in absence of said substance;
d. Determining whether the substance is capable of modulating the amount of MGC4504 mRNA present in said system.

Wherein a substance capable of modulating and preferably increasing the amount of MGC4504 mRNA present in said system is considered a substance active in preventing or treating a disease associated with or caused by a malfunction of the carbohydrate or lipid metabolism.

The derivatives and fragments are as identified above.

A transcriptionally active system is any biochemical or cellular system, which at least has the ability to perform a transcription reaction of a transcription unit. Such systems are well known in the art and comprise cells (e.g. usual laboratory strains or cell lines as well as primary cultures of eukaryotic or prokaryotic cells) as well as in vitro transcription systems or kits (e.g. on basis of cell extracts) which are also commercially available.

The determination of the mRNA amount present In the system can be performed according to techniques well known in the state of the art (etc. direct labelling of the product by means of radioactive or fluorescent labelling or product detection by use of specific primers or probes etc.).

Another example is a method for identifying substances active in the prevention or treatment of a disease associated with or caused by a malfunction of the carbohydrate or lipid metabolism comprising:
a. Contacting a nucleic acid coding for a MGC4504 protein, derivative or fragment thereof with a substance in a translationally active system;
b. Determining the amount of MGC4504 protein, derivative or fragment present in said system in presence of said substance;
c. Determining the amount of MGC4504 protein, derivative or fragment present in said system in absence of said substance;
d. Determining whether the substance is capable of modulating the amount of MGC4504 protein, derivative or fragment present in said system.

Wherein a substance capable of modulating and preferably increasing the amount of MGC4504 protein, derivative or fragment present in said system Is considered to be a substance active in the prevention or treatment of a disease associated with or caused by a malfunction of the carbohydrate or lipid metabolism.

The derivatives and fragments are as identified above.

A translationally active system is any biochemical or cellular system, which at least has the ability to perform a translation reaction of a transcript. Such systems are well known in the art and comprise cells (e.g. usual laboratory strains or cell lines as well as primary cultures of eucaryotic or prokaryotic cells) as well as in vitro translation systems (which are also commercially available, e.g. as kits). For the in vitro translation of a polynucleotide, the polynucleotide is subcloned in a suitable vector, followed by the expression of the polypeptide in suitable buffers and cell extracts (e.g. reticulocyte lysate). Vectors, necessary reagents and protocols with suitable conditions are known in the art and commercially available.

In the context of present invention, the term "polypeptide" refers to a molecule comprising amino acids bound to each other by peptide bonds and which contain at least 10 amino acids coupled to each other in a linear mode. Shorter molecules of this kind are referred to as peptides. The term "protein" refers to molecules comprising at least one polypeptide chain but can also refer to molecules comprising two or more polypeptide chains associated or bound to each other. Thus, the term "protein" comprises the term "polypeptide".

The detection of the MGC4504 protein present in said system can be performed according to techniques well known in the art (e.g. direct radioactive or fluorescent labelling of the translate or the employment of specific antibodies, tagging of the protein and detection of the tag, etc.).

Another aspect of present invention concerns a method for identifying substances active in preventing or treating a disease associated with or caused by a malfunction of the carbohydrate or lipid metabolism comprising:
a. Providing a cell transfected with a nucleic acid vector comprising the MGC4505 promoter or a functional fragment thereof operationally coupled to a reporter gene or a functional fragment thereof;
b. Providing a cell transfected with a control vector which comprises a reporter gene or a functional fragment thereof not being operationally coupled to a functional MGC4504 promoter;
c. Determining the reporter gene activity of the cell according to a) and b) in the absence of a substance;
d. Determining the reporter gene activity in the presence of said substance;

Wherein a substance capable of significantly increasing reporter gene activity according to a) without significantly increasing reporter gene activity of b) (i.e. capable of specifically increasing MGC4504 promoter activity) is considered to be a substance active in the prevention or treatment of a disease associated with or caused by a malfunction of the carbohydrate or lipid metabolism.

A significant increase is any increase higher than the standard deviation, preferably it is at least two times as high as the standard deviation.

The above aspect of present invention is based on a typical reporter gene assay commonly known in the art. To this end, the promoter of choice is inserted into an expression vector suitable for the type of host cell chosen, upstream of the reporter gene of choice in such a way as to allow for an expression of the reporter gene if the promoter is active. The construct is subsequently introduced into the host cell of choice. Suitable methods for transformation or transfection are well known in the art as well as conditions for cell cultivation and detection of reporter gene expression (see e.g. standard literature listed below). Suitable conditions are well known in the art as well as vectors, reporter genes and necessary reagents, which are also commercially available.

A vector is a circular or linear polynucleotide molecule, e.g. a DNA plasmid, bacteriophage or cosmid, by aid of which polynucleotide fragments (e.g. cut out from other vectors or amplified by PCR and inserted in the cloning vector) can specifically be amplified in suitable cells or organisms. Expression vectors enable the heterologous expression of a gene of Interest (e.g. a reporter gene), in the host cell or organism. The type of cell or organism largely depends on the aim and the choice lies within the knowledge of the skilled artisan. Suitable organisms for the amplification of a nucleic acid are e.g. mostly single cell organisms with high proliferation rates, like e.g. bacteria or yeast. Suitable organisms can also be cells isolated and cultivated from multicellular tissues, like e.g. cell lines generated from diverse organisms (e.g. SF9 cells from Spodoptera Frugiperda, etc.). Suitable cloning vectors are known In the art and commercially available at diverse biotech suppliers like, e.g. Roche Diagnostics, New England Biolabs, Promega, Stratagene and many more. Suitable cell lines are e.g. commercially available at the American Type Culture Collection (ATCC).

For the heterologous expression of a protein or polypeptide, the cell can be any prokaryotic or eucaryotic cell capable of being transfected with a nucleic acid vector and of expressing the gene of interest, e.g. a reporter gene. These comprise principally primary cells and cells from a cell culture, preferably an eukaryotic cell culture comprising cells derived either from multicellular organisms and tissue (such as HEK293, RIN-SF, HeLA, CHO, COS, SF9 or 3T3 cells) or single cell organisms such as yeast (e.g. S. pombe or S. cerevisiae), or a prokaryotic cell culture, preferably Pichia or E. coli. Cells and samples derived from tissue can be gained by well-known techniques, such as taking of blood, tissue punction or surgical techniques.

Within the context of present application, the term "transfection" refers to the introduction of a nucleic acid vector into a host cell (either prokaryotic or eukaryotic) and comprises thus the term "transformation".

The transfection can be a stable or transient transfection.

The MGC4504 promoter is a part of the MGC4504 gene able to drive expression of a gene product of interest if introduced into a suitable expression vector upstream of the coding sequence of the gene product. Preferably, the MGC4504 promoter comprises or consists of the sequence according to nucleotides 84361 - 88681 of the sequence according to SEQ ID No.3/NCBI accession number AC020661 [nucleotides 84361 - 88681 correspond to nucleotide positions -4377 / +1]. A functional fragment of the MGC4504 promoter is any fragment of the MGC4504 promoter that is able to drive expression of a gene product of interest if introduced into a suitable expression vector upstream of the coding sequence of the gene product. Preferable fragments comprise functional fragments of the MGC4504 promoter according to nucleotides 84361 - 88681 of SEQ ID No.3.

A reporter gene can be any gene that allows for an easy quantification of its gene product. A vast variety of reporter genes for eukaryotic or prokaryotic hosts as well as detection methods and necessary reagents are known in the art and commercially available. These comprise e.g. the genes of beta Lactamase (lacZ), Luciferase, Green or Blue fluorescent protein (GFP or BFP), DsRed, HIS3, URA3, TRP1 or LEU2 or beta Galactosidase. These genes encode proteins which can be easily detected by means of a visible (colour or luminescent) reaction (e.g. lacZ, Luciferase). These comprise gene-products which can be easily detected by means of a visible (colour or luminescent) reaction or gene-products conferring resistance towards antibiotics like Ampicillin or Kanamycin when expressed. Other reporter gene-products enable the expressing cells to grow under certain conditions like e.g. auxotrophic genes.

A functional fragment of a reporter gene is any fragment of a given reporter gene that allows for an easy quantification of its gene product.

Within the context of the above aspect of present invention the control vector can be any suitable vector which comprises a reporter gene or functional fragment thereof, but wherein reporter gene expression is not driven by a (functional) MGC4504 promoter. This can e.g. mean that the reporter gene or functional fragment thereof is not operationally coupled to a functional MGC4504 promoter (i.e. either totally devoid of an MGC4504 promoter, comprises a non functional MGC4504 promoter or promoter fragment or wherein the coupling of promoter and reporter gene is not functional). This can also mean that the reporter gene or functional fragment thereof is operationally coupled to another promoter than the MGC4504 promoter (e.g. SV40 or another standard promoter). The functional vector and the control vector can also be transfected to the same cell, but In which case the reporter genes need to be different.

Another aspect of present invention concerns the use of a means for the detection of MGC4504 for diagnosing a disease or a predisposition for a disease associated with or caused by a malfunction of the carbohydrate or lipid metabolism in an isolated sample of an individual.

The diagnosis can e.g. comprise
1. The detection of the amount of MGC4504 (protein or mRNA) present in the isolated sample; wherein a lowered amount in comparison to a reference sample indicates the predisposition or the disease.
2. The detection of the activity of MGC4504 present in the isolated sample; wherein a lowered activity in comparison to a reference sample is indicative of the predisposition or the disease.
3. The detection of a mutation within the MGC4504 protein/gene/coding sequence/promotor etc. leading to a lowered amount or a lowered activity or malfunction of MGC4504, wherein the detection of the mutation is indicative of the predisposition or the disease.

Wherein the reference sample can for example be an isolated sample having an average activity or amount of MGC4504 and/or a sample of a donor not having a disease or a predisposition for a disease associated with or caused by a malfunction of the carbohydrate or lipid metabolism.

The means to detect MGC4504 protein or polypeptide can be a means able to specifically detect either wildtype MGC4504 protein/polypeptide and can also be a means to detect specifically MGC4504 protein/polypeptide harbouring one or more mutations regarding the size or the amino acid sequence in comparison to a wild type polypeptide/protein: The means comprises or is an antibody able to specifically detect MGC4504 protein, e.g. for use in immunohistological or immunohistochemical techniques (e.g. detection of MGC4504 protein or certain mutations thereof in histological tissue sections or MGC4505 protein immobilized on suitable carriers like membranes, chips, ELISA plates etc.)

The means to detect MGC4504 nucleic acid can e. g. be a means to detect MGC4504 mRNA /cDNA or genomic DNA, either wildtype or also harbouring one or more mutations regarding their length or their nucleic acid sequence in comparison to a wild type MGC4504 nucleic acid. The means can e.g. be a means to specifically detect and/or quantify MGC4504 mRNA and comprises or is a specific MGC4504 nucleic acid probe or a primer set capable of amplifying MGC4504 DNA or, e.g. for use in PCR sequencing (for the detection of Mutations in the nucleotide sequence) or capable of amplifying MGC4504 cDNA, e.g. for use in RT PCR (for the detection and/or quantification of MGC4504 mRNA expression). Another means can e.g. be a nucleic acid probe able to specifically hybridize to MGC4504 mRNA or cDNA under standard conditions, e.g. for use in Northern Blot or Chip hybridization techniques.

The term wild type refers to the genotype or phenotype that is found in nature or in the standard laboratory stock for a given organism. According to one preferred embodiment, the wildtype sequences of MGC4504 are the sequences according to SEQ ID No. 1,2,3 or 14.

The design and synthesis of suitable primers is well known in the art (see also above). According to a preferred embodiment of present invention, the means is a primer set for the amplification of MGC4504 nucleic acid, and preferably a set of primers comprising at least one of the primers according to SEQ ID No. 8 and/or 9.

According to a further preferred embodiment of present invention, the means is a probe for the detection of MGC4504 nucleic acid and preferably a probe having the sequence according to SEQ ID No.10. The design and synthesis of suitable probes is well known in the art (see also standard literature below).

According to yet another preferred embodiment of present invention, the means is an antibody for the specific detection of MGC4504 protein or polypeptide. The preparation of suitable antibodies or functional fragments thereof is well known in the art as well, e.g. by Immunizing a mammal, for example a rabbit, with MGC4504 protein or a fragment thereof, where appropriate in the presence of, for example, Freund's adjuvant and/or aluminum hydroxide gels (see, for example, Diamond, B.A. et at. (1981) The New England Journal of Medicine: 1344-1349). The polyclonal antibodies which are formed in the animal as a result of an immunological reaction can subsequently be isolated from the blood using well-known methods and, for example, purified by means of column chromatography. Monoclonal antibodies can, for example, be prepared in accordance with the known method of Winter & Milstein (Winter, G. & Milstein, C. (1991) Nature, 349, 293-299). Suitable procedures to produce monoclonal antibodies are well known in the art as well (see e.g. literature for standard methods listed below). In the context of present invention, the term antibody or antibody fragment comprises also antibodies or antigen-binding parts thereof, which have been prepared recombinantly and, where appropriate, modified, such as chimaeric antibodies, humanized antibodies, multifunctional antibodies, bispecific or oligospecific antibodies, single-stranded antibodies and F(ab) or F(ab)₂ fragments (see, for example, EP-B1-0 368 684, US 4,816,567, US 4,816,397, WO 88/01649, WO 93/06213 or WO 98/24884).

Isolated samples comprise any kind of biological samples isolated from an Individual, e.g. cells, preparations, sections or parts of tissue or organs (e.g. muscle, pancreas, brain, blood, liver, spleen, kidney, heart, blood vessels, etc.). Isolated samples can be gained by well-known techniques, such as taking of blood, tissue punction or surgical techniques. The preparation of cell- or tissue extracts is well known to the person of skill in the art (see e.g. also the standard literature listed below).

The present invention discloses the use of a means for the detection of MGC4504 for the manufacture of a diagnostic kit for the identification of a disease or a predisposition for a disease associated with or caused by malfunctions of the carbohydrate and/or lipid metabolism.

Another aspect of present invention concerns a method of diagnosing a predisposition for or a malfunction of the carbohydrate and/or lipid metabolism and comprises analyzing the amount of MGC4504 present in an isolated sample of an individual, wherein a lowered MGC4504 amount in comparison to one or more reference samples is indicative of the predisposition or malfunction.

Within the context of the different aspects of present invention, the individual can be any living being and is preferably a mammal and more preferably a human being.

The analysis of the amount of MGC4504 can for example be an analysis of the mRNA amount present in the sample, e.g. by means of Northern Blot analysis, Chip analysis (like cDNA microarrays) or quantitative RT PCR. The analysis can also be an analysis of the protein amount present in the sample, e.g. by means of techniques employing specific antibodies like ELISA, Western Blotting, Chip techniques (like protein microarrays), etc.

Yet another aspect of present invention concerns a method of diagnosing a disease or a predisposition for a disease associated with or caused by a malfunction of the carbohydrate and/or lipid metabolism comprising analyzing an isolated sample of an individual for mutations of MGC4504 in comparison to a reference sequence of MGC4504, wherein the presence of a mutation is indicative of the disease or the predisposition.

Mutations, i.e. deviations from the most frequently found genotype (also called the wildtype with respect to a gene), are well known in the art. These comprise, among others, single nucleotide polymorphisms (SNPs, i.e. variants of a given nucleotide sequence with exchanges at single nucleotide positions), polymorphisms of lengths, deletions, inversions etc. Genetic mutations can effect gene expression or protein function and they can therefore be implicated in the onset and predisposition for certain diseases. Due to the implication of MGC4504 in pathological processes underlying diabetes found out by the inventors, mutations of the MGC4504 gene, and especially those affecting MGC4504 expression and/or function and/or activity, are bound to have a high impact on diseases associated with or caused by malfunctions of the carbohydrate and/or lipid metabolism.

The diagnostic methods can be performed according to any known method for detecting MGC4504, e.g. by use of one of the above means of detecting MGC4504 in a sample. The mutations can e.g. be analyzed on a genomic level, e.g. by means of the molecular biological analysis of the MGC4504 gene (such as Southern Blotting, genomic sequencing techniques, mass spectroscopic analysis of the sequence, DNA Microarrays etc.). The mutations can also be analyzed on the level of the expressed mRNA or protein, e.g. by means of sequencing of the cDNA (e.g. on the basis of RT PCR) or by protein sequencing techniques or usage of specific antibodies.

The reference sample can be a sample as defined above.

A reference sequence can e.g. be the MGC4504 wild type sequence (see e.g. above) and/or the MGC4504 sequence found in most individuals not having the above disease or predisposition therefore (in this case, the reference sequence can also be sequence not present in biggest part of the population but in biggest part of population not having the above disease or predisposition therefore; in this case the wild type sequence is associated with an increased risk for developing said disease). According to a preferred embodiment the reference sequence is one of the wild type sequences of MGC4504 (see above).

According to a preferred embodiment of said aspect of present invention, the mutations are mutations leading to a lowered amount of MGC4504 and/or activity or to a complete lack of MGC4504 and/or a total lack of activity.

Another aspect of present invention concerns a method of diagnosing a predisposition for or a malfunction of the carbohydrate and/or lipid metabolism and comprises analyzing the activity of MGC4504 present in an isolated sample of an individual, wherein a lowered MGC4504 activity in comparison to one or more reference samples is indicative of the predisposition or malfunction.

The activity can be any protein activity implied in one of the functions of MGC4504 as defined above. The activity can be measured directly or Indirectly (e.g. by analyzing the intensity or extent of one or more of the functions of MGC4504).

Another aspect of present invention concerns a kit for the identification of a disease or predisposition for a disease associated with or caused by malfunctions of the carbohydrate or lipid metabolism comprising at least one means for the detection of MGC4504.

In the context of the present invention, a kit (also known as "kit of parts") is understood to be any combination of one or more of the components as defined in the claims. which are combined, coexisting spatially, to a functional unit, and which can contain further components.

In the context of present invention, the kit comprises at least a means for the detection of MGC4504, suitably together with suitable buffers and/or reagents for detecting MGC4504 and/or sample preparation, and optionally a handling manual for performing the respective detection technique.

The means are means as defined by the claims and preferably comprises or is a specific MGC4504 nucleic acid probe or a primer set capable of amplifying a MGC4504 nucleic acid as outlined in the claim 9.

The present invention discloses, without including, a method of adapting the medication for the treatment or prevention of a disease associated with or caused by malfunctions of the carbohydrate and/or lipid metabolism, wherein an isolated sample of an individual is analysed for a mutation of MGC4504 in comparison to a reference sequence and/or a decreased amount and/or activity of MGC4504 in comparison to a reference sample, wherein the dosage is adapted if a mutation and/or a decreased amount and/or activity of MGC4504 is present in the sample.

The adaptation of the medication can e.g. be the adaptation of the kind of medication applicable for the individual, i.e. for example the determination whether the individual can be at all administered or effectively treated with a substance (e.g., an agonist, antagonist, peptidomimetic, protein, peptide, nucleic acid, small molecule, or other drug candidate) or a specific class of substances (e.g., substances of a type which directly increase MGC4504 protein activity or substances belonging to a subset of chemical compounds with a common structural motif) to treat or prevent a disease associated with or caused by malfunctions of the carbohydrate and/or lipid metabolism. According to another example the adaptation of the medication can be the adaptation of the dosage of a given medlcament/substance or class of substances.

Substances which have a stimulatory or inhibitory effect (and preferably a stimulatory effect) on MGC4504 expression or activity (e.g., MGC4504 gene expression) as identified by a screening assay can be used for preparing a pharmaceutical which is useful for the treatment or prevention of a disease associated with or caused by malfunctions of the carbohydrate and/or lipid metabolism (e.g., disorders involving cells or tissues in which MGC4504 is expressed, such as myocytes associated with aberrant or a lack of MGC4504 activity). In conjunction with such treatment, the pharmacogenomics (i.e. the study of the relationship between an individual's genotype and that individual's response to a foreign compound or drug) of the individual may be considered. Differences in metabolism of therapeutics can lead to severe toxicity or therapeutic failure by altering the relation between dose and blood concentration of the pharmacologically active drug. Thus, the pharmacogenomics of the individual permits the selection of effective agents (e.g., drugs) for prophylactic or therapeutic treatments based on a consideration of the individual's genotype. Such pharmacogenomics can further be used to determine appropriate dosages and therapeutic regimens.
Accordingly, the activity of MGC4504 protein, expression of MGC4504 nucleic acid, or mutation content of MGC4504 genes in an individual can be determined to thereby select appropriate agent(s) for therapeutic or prophylactic treatment of the individual.

Yet another aspect of present invention concerns MGC4504 or a functional fragment or derivative thereof or a composition comprising MGC4504 or a functional fragment or derivative thereof for use in a method for the treatment or prevention of a disease associated with or caused by a malfunction of the carbohydrate or lipid metabolism.

To this and, MGC4504 or fragments or derivatives thereof may either be used in the form of a polypeptide or peptide or an oligo- or polynucleotide. Useful are suitable modifications or additives for ensuring or facilitating its targeting to the site of need and its entering the cell or its stability. On the other hand, a local application, such as a subcutaneous, subdermal or intramuscular injection, etc. or the like is also possible for ensuring its targeting to the site of need. Other useful additives include salts, buffers or the like for its stabilization, etc.

The MGC4504 polynucleotide or functional fragment or derivative thereof can e.g. be put into a suitable vector that ensures its intracellular expression or also its targeting into a cell. A cell type specific expression can be ensured using appropriate promoters/enhancers of cell type or tissue-specific genes, which are known in the art. The use of MGC4504 oligonucleotides is also possible.

For the production of a medicament the active ingredients are usually formulated with suitable additives or auxiliary substances, such as physiological buffer solution, e.g. sodium chloride solution, demineralized water, stabilizers, such as protease or nuclease inhibitors, preferably aprotinin, ε-aminocaproic acid or pepstatin A or sequestering agents such as EDTA, gel formulations, such as white vaseline, lowviscosity paraffin and/or yellow wax, etc. depending on the kind of administration.

Suitable further additives are, for example, detergents, such as, for example, Triton X-100 or sodium deoxycholate, but also polyols, such as, for example, polyethylene glycol or glycerol, sugars, such as, for example, sucrose or glucose, zwitterionic compounds, such as, for example, amino acids such as glycine or in particular taurine or betaine and/or a protein, such as, for example, bovine or human serum albumin. Detergents, polyols and/or zwitterionic compounds are preferred.

The physiological buffer solution preferably has a pH of approx. 6.0-8.0, preferably a pH of approx. 6.8-7.8, in particular a pH of approx. 7.4, and/or an osmolarity of approx. 200-400 milliosmol/liter, preferably of approx. 290-310 milliosmol/liter. The pH of a medicament is in general adjusted using a suitable organic or inorganic buffer, such as, for example, preferably using a phosphate buffer, tris buffer (tris (hydroxymethyl)aminomethane), HEPES buffer ([4-(2-hydroxyethyl)piparazino] ethanesulphonic acid) or MOPS buffer (3-morpholino-1-propanesulphonic acid). The choice of the respective buffer in general depends on the desired buffer molarity. Phosphate buffer is suitable, for example, for injection and infusion solutions (see also Remington's Pharmaceutical Sciences, 17th Edition, 1985 (for physiologically tolerable salts (anorganic or organic), see esp. p. 1418).

The medicament can be administered in any suitable conventional manner, e.g. by means of oral dosage forms, such as, for example, tablets or capsules, by means of the mucous membranes, for example the nose or the oral cavity, in the form of dispositories implanted under the skin, by means of injections, infusions or gels which contain the medicaments according to the invention. It is further possible to administer the medicament locally in order to treat the particular disease as described above, if appropriate, in the form of liposome complexes. Furthermore, the treatment can be carried out by means of a transdermal therapeutic system (TTS), which makes possible a temporally controlled release of the medicaments. TTS are known for example, from EP 0 944 398 A1, EP 0 916 336 A1, EP 0 889 723 A1 or EP 0 852 493 A1.

Administration should suitably be performed in a way that allows for targeting of MGC4504 to the site of action (e.g. muscular, brain or pancreatic tissue), e.g. by systemic administration of MGC4504 derivatives or formulations targeting themselves to site of need or local (e.g. intramuscular) application of formulation containing MGC4504 or fragments or derivatives thereof.

Injection solutions are in general used if only relatively small amounts of a solution or suspension, for example about 1 to about 20 ml, are to be administered to the body. Infusion solutions are in general used if a larger amount of a solution or suspension, for example one or more litres, are to be administered. Since, in contrast to the infusion solution, only a few milliliters are administered in the case of injection solutions, small differences from the pH and from the osmotic pressure of the blood or the tissue fluid in the injection do not make themselves noticeable or only make themselves noticeable to an insignificant extent with respect to pain sensation. Dilution of the medicament according to the invention before use is therefore in general not necessary. In the case of the administration of relatively large amounts, however, the medicament according to the invention should be diluted briefly before administration to such an extent that an at least approximately isotonic solution is obtained. An example of an isotonic solution is a 0.9% strength sodium chloride solution. In the case of Infusion, the dilution can be carried out, for example, using sterile water while the administration can be carried out, for example, via a so-called bypass.

A further aspect of present invention concerns the in vitro-use of a cell heterologously expressing MGC4504 for the identification of substances active in the treatment or prevention of diseases associated with or caused by malfunctions of the carbohydrate or lipid metabolism. According to a preferred embodiment, the cell is a transgenic cell.

According to another aspect of present invention a transgenic non-human animal heterologously expressing MGC4504 can be used for the identification of substances active in the treatment or prevention of diseases associated with or caused by malfunctions of the carbohydrate or lipid metabolism.

A transgenic cell/animal is a cell/animal that carries in its genome a transgene in addition to its normal set of genes. The production of transgenic cells and animals as well as the necessary vector constructs is well known in the art (for an overview see e.g. Gene targeting: A Practical Approach, 2nd Ed., Joyner AL, ed. 2000. IRL Press at Oxford University Press, New York; Manipulating the Mouse Embryo: A Laboratory Manual. Nagy, A, Gertsenstein, M., Vintersten, K., Behringer, R., 2003, Cold Spring Harbor Press, New York; Transgenic Animal Technology: A Laboratory Handbook, Pinkert CA, ed., 1994, Academic Press., New York) and their production is also offered as a commercial service by several suppliers e.g. (The Jackson Laboratory, Bar Harbor, Maine, USA).

According to the different aspects of present invention, the transgene is MGC4504 or a fragment or derivative thereof, either functional or with an impaired or abolished function.

According to another preferred embodiment of present invention, a modified cell, having a lower MGC4504 activity as compared to its unmodified state, is used. This way, it can be tested, if the chemical compounds to be tested are able to enhance or restore the lowered or totally abolished MGC4504 activity.

The modification can be any type of modification (stable or transient, preferably stable), that leads to a decrease of MGC4504 activity (i.e. its ability to interact with other molecules, its ability to increase insulin sensitivity of cells etc.), MGC4504 transcript steady state level (i.e. by activation of MGC4504 transcription or transcript stabilization) or MGC4504 protein steady state level (i.e. by activation of MGC4504 translation or its posttranslational processing; by modulation of MGC4504 posttranslational modification or by activation of its stabilization or by inhibition of its degradation). This can for example be achieved by using dominant negative mutants of MGC4504, antisense oligonucleotides, RNAi constructs of MGC4504, by generating functional or genomic MGC4504 knock outs (which can e.g. be inducible) or other suitable techniques known within the state of the art. For an overview of the above techniques, see for example: Current protocols in Molecular biology (2000) J.G. Seidman, Chapter 23, Supplement 52, John Wiley and Sons, Inc.; Gene Targeting: a practical approach (1995), Editor: A.L. Joyner, IRL Press; Genetic Manipulation of Receptor Expression and Function, 2000; Antisense Therapeutics, 1996; Scherr et al, 2003.

According to another aspect, the present invention concerns the use of a MGC4504 knock-out cell in vitro or of a non-human knockout animal for the identification of substances active in the treatment or prevention of diseases associated with or caused by malfunctions of the carbohydrate or lipid metabolism. The knockout can be a genomic knockout or a functional knockout. Suitable cell lines for the generation of knockouts are well known in the state of the art and protocols for the generation of knockouts comprise for example those disclosed in Current protocols in Molecular Biology (2000) J.G. Seidman, Chapter 23, Supplement 52, John Wiley and Sons, Inc; or Gene targeting: A Practical Approach, 2nd Ed., Joyner AL, ed. 2000. IRL Press at Oxford University Press, New York; Manipulating the Mouse Embryo: A Laboratory Manual. Nagy, A, Gertsenstein, M., Vintersten, K., Behringer, R., 2003, Cold Spring Harbor Press, New York. Moreover, knockouts of genes of interest are offered as a service by commercial suppliers (e. g. The Jackson Laboratory, Bar Harbor, Maine, USA).

Another aspect of present invention concerns a high throughput screen comprising a method according to one of the above novel methods for the identification of active substances (such methods are also called "assays").

Analytical methods or analytical systems, so-called assays, which are used to measure the activity or concentration of defined target molecules (so-called targets, mostly proteins or nucleic acids) as parameter for the effectiveness of a potential pharmaceutical compound, are well known in the state of the art. Assays comprise for example biochemical analytical methods or systems using isolated or partly isolated components that are put together to a reaction mixture within a defined space and time, in which the effectiveness of the potential pharmaceutical compounds can be tested. Other examples of assays comprise biochemical analytical methods or systems, in which the activity of the target molecule and the effectiveness of a potential to influence this activity, can be determined within a cell.

An assay can be any type of analytical method or system to monitor a biological process (see e.g. the above analytical methods). Suitably, molecular cascades and mechanisms representing parts of physiological metabolic pathways but also of pathological conditions are reproduced in cellular or biochemical (in vitro) systems. The pharmacological activity of a potential pharmaceutical compound can thus be determined according to its capability of interfering with or modulating these cascades or mechanisms.

For the use in drug screening, especially the high throughput screening for novel pharmaceutical compounds, the assay needs to be reproducible and is preferably also scalable and robust. In the scope of present invention, high throughput screen means, that a method according to present invention is performed in a very small scale, e.g. on 96, 386 or 1536 well plates in samples of very small volume in the range of few milliliters down to few nanoliters or even less. Thus, a very large amount of samples can be analyzed in a short time. High throughput screening mostly comprises the screening of up to approximately 500.000 different compounds for certain ability by means of one single assay. The assay is preferably suitable for high throughput screening of chemical substances for their ability of modulating the activity of the target molecule under investigation. The type of assay depends e.g. on the type of target molecule used (e.g. polypeptide or polynucleotide) and the "read out", i.e. the parameter, according to which the activity of the target molecule is determined (see below).

Different types of assays are commonly known in the state of the art and commercially available from commercial suppliers.

Suitable assays for different purposes encompass radio isotopic or fluorescent assays, for example fluorescence polarization assays (for measuring the interaction of a labelled member with a non-labelled member (e.g. the interaction of labelled protein receptors with their unlabeled ligands).

More examples include cell based assays, wherein a cell line stably (inducible or not; chromosomal or episomal) or transiently expresses a recombinant protein of interest. These assays comprise e.g. reporter gene assays, wherein the regulation of a certain promoter or a signal transduction pathway of a member of a signal transduction cascade is measured according to the activity of a reporter enzyme, the expression of which is under the control of said certain promoter. For this type of assay, a recombinant cell line has to be constructed containing the reporter gene under the control of a defined promoter that is to be investigated itself or that is regulated by ths signalling cascade under investigation. Suitable reporter enzymes are commonly known within the state of the art and comprise different types of luciferase or B-galactosidase. Suitable cell lines depend on the aim of the assay but comprise mostly cell lines that are easy to transfect and easy to cultivate, such as, e.g. HEK293, HeLA, COS, CHO, NIH-3T3, etc.

Assays for measuring the intracellular ion level comprise e.g. FLIPR (fluorometric imaging plate reader, commercially available from Molecular Devices) assays, wherein an argon laser light source combined with a cooled CCD camera allows for parallel measurements in 384 well plates transient ion signals (such as Ca²⁺, etc) within cells (e.g. neuronal cells or other cells (e.g. cells recombinantly or naturally expressing certain ion channels). FLIPR assays allow e.g. for monitoring of intracellular calcium using certain fluorochromes or detecting membrane potential changes or monitoring of membrane polarization using specific FLIPR assay kits. For the monitoring of other intracellular ions, e.g. zinc or sodium, other dyes known In the state of the art can be used. Other types of assays and other types of read outs are commonly known to persons with skills in the art.

For the measurement of cAMP levels, e.g. ALPHAScreen^{™}, fluorescence polarization or HTRF technology are suitable.

For the determination of ion channel activity (which control e.g. intracellular ion concentrations and can thus be employed for measurement of intracellular ion concentrations) e.g. membrane potential sensitive assays and dyes can be used.

For measurement of GPCR activity, e.g. cAMP measurement, for example by means of the AlphaScreen^{™} cAMP detection system by Packard Bioscience, Ca2+ mobilization-assays or reporter gene assays are suitable.

For determination of protein phosphorylation e.g. kinase activity, fluorescence polarization assays are suitable, e.g. commercially available by Panvera; moreover HTRF (homogeneous time resolved fluorescence, Cis Bio), LANCE assays (Perkin Elmer Life Science) or the amplified luminescence proximity homogeneous assay (ALPHAScreen^{™} by Packard BioScience) are applicable. Other types of assays and other types of "read out" are well known in the state of the art.

According to one preferred embodiment of the different aspects of present invention, MGC4504, the derivative or fragment thereof can be used as an isolated molecule.

In the context of this invention, the term "isolated molecule", especially with respect to MGC4504, refers to MGC4504 polynucleotides or polypeptides purified from natural sources as well as purified recombinant molecules (wherein the term purified comprises a partial purification as well as a complete purification).

The preparation of recombinant polypeptide or polynucleotide molecules and the purification of naturally occurring molecules from cells or tissue, as well as the preparation of cell- or tissue extracts is well known to the person of skill in the art (see e.g. also the standard literature listed below).

These comprise e.g. amplifying polynucleotides of desired length via the polymerase chain reaction (PCR) on the basis of the published genomic or coding polynucleotide sequences and the subsequent cloning of the produced polynucleotides in host cells (see e.g. standard literature listed below).

The PCR is an in vitro technique that enables the specific amplification of sequence stretches having nucleotide stretches of known sequence in their 5' and 3' vicinit. For amplifying the sequence of choice, short single-stranded DNA molecules ("primers") are used, which are complementary to the sequence stretches framing the polynucleotide sequence to be amplified. The polynucleotide template can either be DNA or RNA. By choosing defined sequences of incubation steps at defined temperatures and of defined time Intervals, that are repeated periodically, the polynucleotide of interest is amplified exponentially.

Suitable primers can be generated by means of chemical synthesis according to well-known protocols. Such primers are also commercially available by different suppliers, such as MWG Biotech etc.

DNA and RNA templates, also cDNA templates can be generated by means of well known standard procedures (such as DNA templates cloned by aid of cloning vectors; the preparation of genomic DNA or RNA from culture cells, tissue, etc or preparation of cDNA from such sources of RNA, etc., see, e.g. the below standard literature) and can also be purchased from commercial suppliers, such as Promega and Stratagene, etc. Suitable buffers and enzymes as well as reaction protocols for performing the PCR are known in the art and commercially available as well. The reaction product can be purified by known procedures (e.g. gel purification or column purification).

Another method of generating isolated polynucleotides Is the cloning of a desired sequence and its subsequent complete or partial purification by means of standard methods. For generating isolated polypeptides, the polynucleotides are cloned into expression vectors and the polypeptides are expressed in suitable host organisms, preferably single cell organisms like suitable strains of bacteria or yeast, followed by the subsequent complete or partial purification of the polypeptide.

The methods according to present invention can e.g. be performed as a biochemical or cellular assay.

MGC4504 can be derived from any sequence available that allows for its specific purpose according to one of the different aspects of the present invention. Preferably, MGC4504 is human MGC4504.

According to one preferred embodiment, MGC4504 is used as a polynucleotide. Preferably, the polynucleotide comprises or consists of
a. SEQ ID No. 1
b. A sequence capable of hybridising with a sequence according to a) at conditions of high stringency and coding for a polypeptide with MGC4504 function;
c. A sequence derived from a sequence according to a) or b) due to the degeneracy of the genetic code and is coding for a polypeptide with MGC4504 function;
d. A fragment of one of the sequences according to a), b) or c), coding for a polypeptide with MGC4504 function; and coding for a polypeptide comprising or consisting of the sequence according to SEQ ID No. 2;
wherein the MGC4504 function as indicated in b. to d. is as indicated above.

A preferred polynucleotide fragment is the fragment according to SEQ ID No. 14.

The term "stringency" describes reaction conditions that influence the specificity of hybridization or annealing of two single stranded nucleic acid molecules.

A nucleic acid molecule can hybridize to another nucleic acid molecule when the single stranded forms of both molecules can anneal under suitable reaction ("annealing" or hybridization) conditions (dependent on temperature and ionic strength of the surrounding medium) to form a new double stranded nucleic acid molecule. Hybridization requires that the two annealing nucleic acid molecules comprise complementary sequences. Depending on the selected annealing conditions, the stringency conditions, mismatches between the bases are possible without preventing double strand formation.

Stringency, and thus specificity of a reaction depends, inter alia, of the temperature and buffer-conditions used for a reaction: Stringency, and thus specificity, can e.g. be increased by increasing the reaction temperature and/or by lowering the ion strength of the reaction-buffer. Suitable conditions of stringency for the hybridization of nucleic acids depend on the length, the type of nucleic acid and their degree of complementarity. The variables are known in the state of the art. The higher the degree of similarity or homology between two annealing nucleotide sequences, the higher is the melting temperature for hybridization products of nucleic acids with those sequences. The relative stability of nucleic acid hybridization is dependent according to the type of the single stranded nucleic acids forming the double strand: RNA:RNA>DNA:RNA>ONA:DNA. For hybridization products of more than 100 nucleotides in length, equations for calculating the melting temperature are known In the art. For shorter hybridization products (e.g. oligonucleotides) the calculation of the melting temperature is dependent on the length, wherein mismatches become more important.

Conditions of low stringency (and thus low reaction and hybridization specificity) exist for example, if a hybridization is performed at room temperature in 2xSSC-solulion. Conditions of high stringency comprise e.g. a hybridization reaction at 68°C in 0,1xSSC and 0,1% SDS solution.

In the context of present invention the term "hybridizing under conditions of stringency" refers to conditions for the performance of the hybridization reaction and the following washing procedure, at which nucleotide sequences with at least 50, 55, 60, 65, 70 and preferably 75% or more complementarity typically remain hybridized. The choice of such conditions for a given set of nucleic acids lies within the skill of the average artisan, and suitable protocols can be found in well known literature for standard methods like, for example, "Current Protocols in Molecular Biology", John Wiley & Sons, N.Y. (1989), 6.3.1 to 6.3.6 (see also literature listed below).

Hybridization under conditions of stringency within the different aspects of present invention is preferably understood to be:
1) Hybridizing a labelled probe with a nucleic acid sample to be analyzed at 65°C, or in the case of oligonucleotide probes, at 5°C below the annealing or melting temperature of the duplex consisting of oligonucleotide and sample (annealing and melting temperature are in the following understood to be synonyms) over night in 50mM Tris pH 7,5, 1M Nacl, 1% SDS, 10% Dextran Sulfate, 0,5 mg/ml denatured salmon or hering sperm DNA.
2) Washing for 10 minutes in 2xSSC at room temperature.
3) Washing for 30 minutes in 1xSSC/0.1%SDS at 65°C (or in the case of oligonucleotides: 5°C below the annealing temperature).
4) Washing for 30 minutes in 0,1 x SSC/0,1%SDS at 65°C (or in the case of oligonucleotides: 5°C below the annealing temperature).

Oligonucleotides are polynucleotide and preferably DNA-fragments having a length of 15 to 30, preferably 20 nucleotides. The annealing temperature is determined according to the formula Tm=2x (number of A+T) + 4x (number of G+C)°C.

For preparing a 2xSSC or a 0,1xSSC (or any other kind of SSC dilution), e.g. a 20x SSC solution is diluted accordingly. 20xSSC consists of 3M NaCl/0,3 M Na-Citrate x 2H₂O.

Before performing of a hybridization reaction, the polynucleotides are, if wanted after performing electrophoretic separation (then: Southern Blot (DNA) or Northern Blot (RNA)) or without electrophoretic separation (then: slot or dot Blot), transferred to a suitable membrane, e.g. a nylon or nitrocellulose membrane. Hybridization is performed using a suitably labelled probe. Suitable labelling techniques are e.g. radioactive labelling or labelling using fluorescence dyes. The probe is a single stranded polyribo- or polydesoxyribonucleotide being single stranded naturally or being usually double stranded and having been made single stranded by denaturation. This probe binds to the DNA or RNA sample (which is also in single stranded state) by means of base pairing.

According to a preferred embodiment of the different aspects of present invention the conditions of stringency are conditions of high stringency.

According to another embodiment, MGC4504 is used as a polypeptide. Preferably the polypeptide is encoded by one of the following sequences:
a. SEQ ID No. 1 or 14;
b. A sequence capable of hybridizing with the sequence according to a) at conditions of high stringency and coding for a polypeptide with MGC4504 function;
c. A sequence derived from a sequence according to a) or b) due to the degeneracy of the genetic code and coding for a polypeptide with MGC4504 function;
d. A fragment of one of the sequences according to a), b) or c), coding for a polypeptide with MGC4504 function;
wherein the MGC4504 function as indicated in b. to d. is as indicated above.

According to yet another preferred embodiment, the polypeptide comprises or consists of one the sequences according to SEQ ID No 2.

The disease associated with or caused by a malfunction of the carbohydrate or lipid metabolism is a malfunction of the glucose metabolism, obesity, dislipidemia or the metabolic syndrome or diabetes or any other malfunction of insulin action and more preferably type 1 or type 2 diabetes mellitus or insulin resistance.

In the following, the invention is described in more detail by means of different examples without meaning to be limited by these examples.

### Legend to the Figures

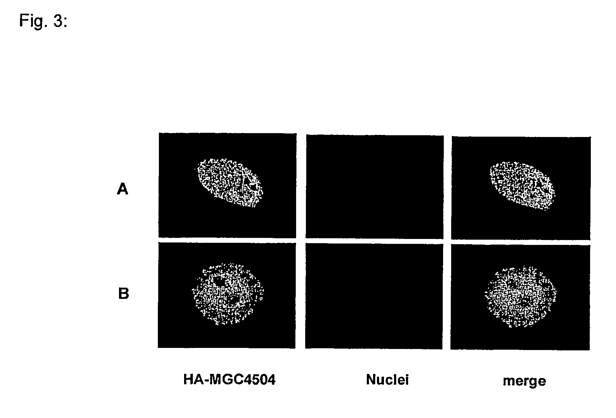

Table 1:
   Affymetrix Gene Chip^{®} Analysis of MGC4504 gene expression in merged replicates of lean insulin sensitive controls and obese insulin resistant or diabetic ZDF rats.
Table 2:
   Quantitative real-time PCR of RNA samples from lean insulin sensitive controls and obese insulin resistant or diabetic ZDF rats.
Table 3:
   Affymetrix Gene Chip^{®} analysis of MGC4504 gene expression in a diet-induced model of type 2 diabetes
Figure 1:
   MGC4504 gene expression levels in a selected subset of different human tissues.
Figure 2:
   Stable expression of HA-MGC4504 in L6GLUT4myc myoblasts.
Figure 3:
   Subcellular distribution of MGC4504 in cytoplasm and nucleus of transiently transfected L6GLUT4myc (Figure 3A) and RIN-5F (Figure 3B) cells..
Figure 4:

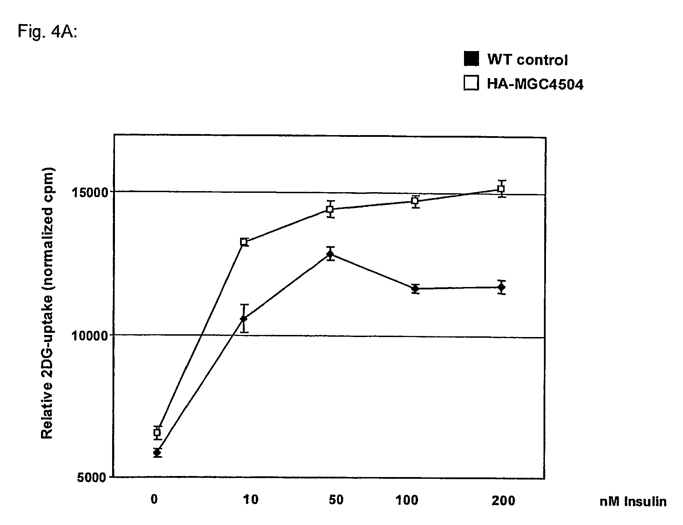

   Figure 4A: 2-Deoxyglucose uptake assay and increased insulin sensitivity of L6GLUT4myc HA-MCG4504 cells.

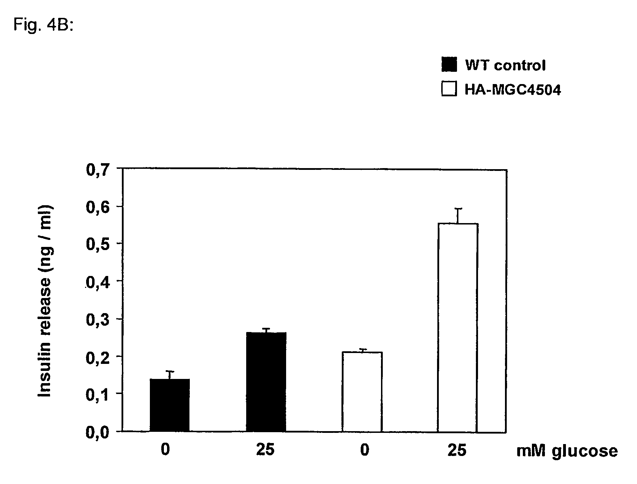

   Figure 4B: Insulin secretion assay and increased basal and glucose stimulated insulin secretion of RIN-5F HA-MGC4504 cells.
Figure 5:
   Coomassie staining of different samples harvested during expression and purification of soluble GST-MGC4504 protein.
Figure 6:

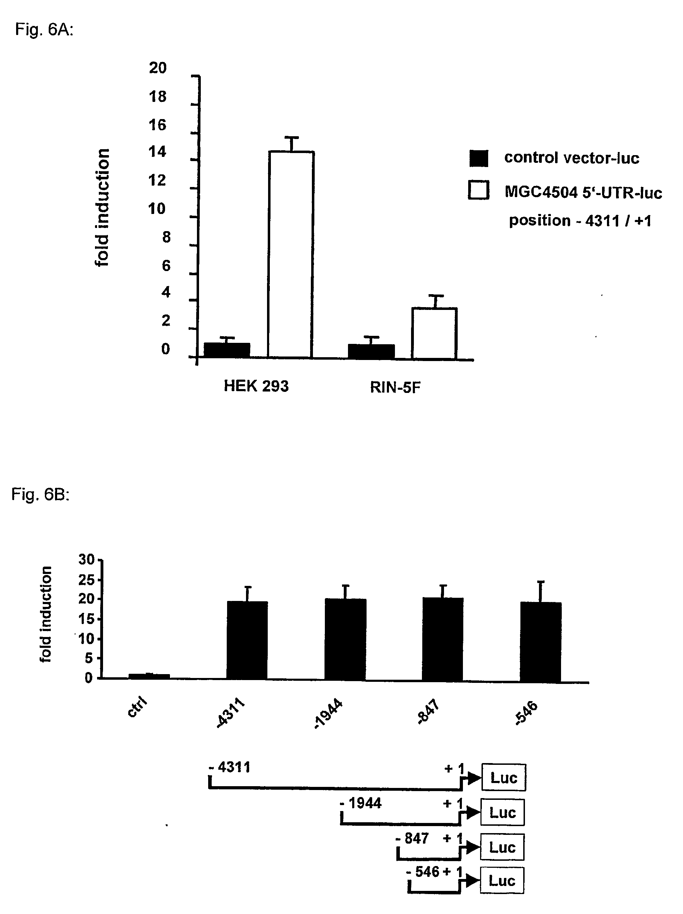

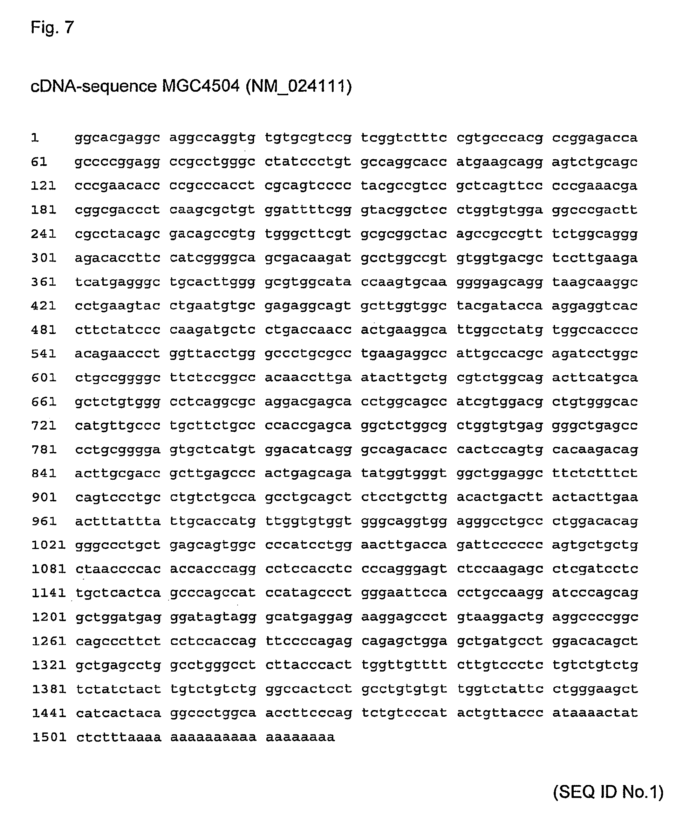

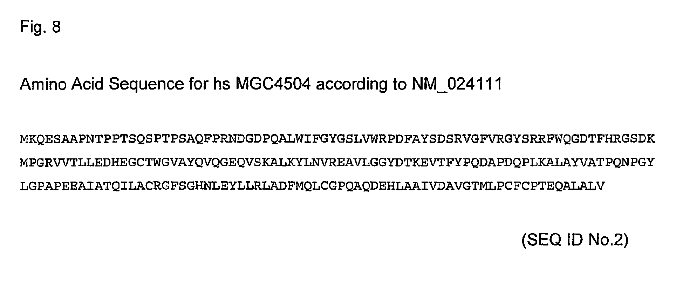

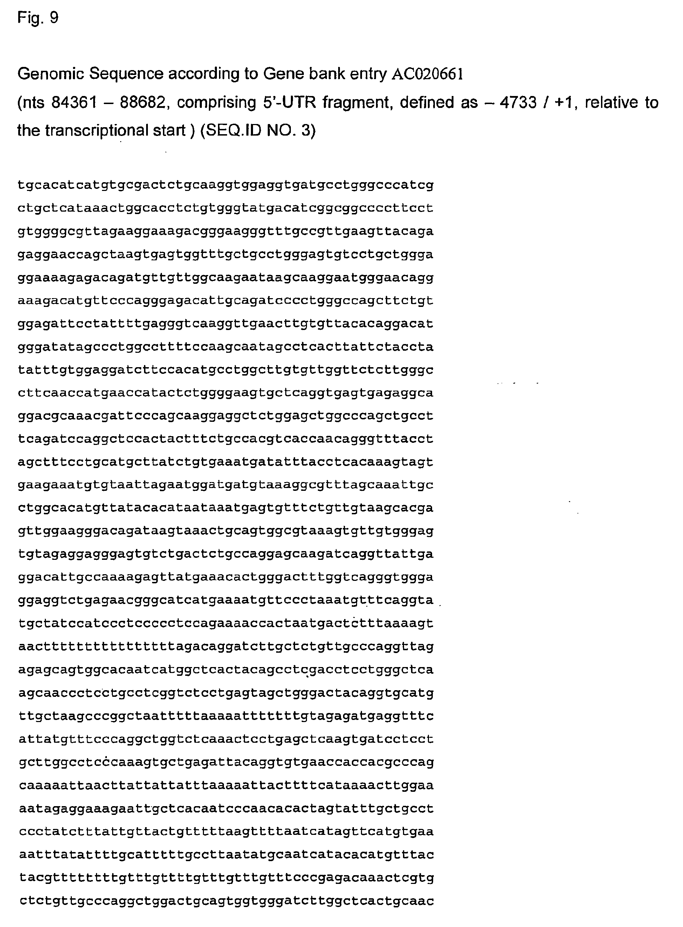

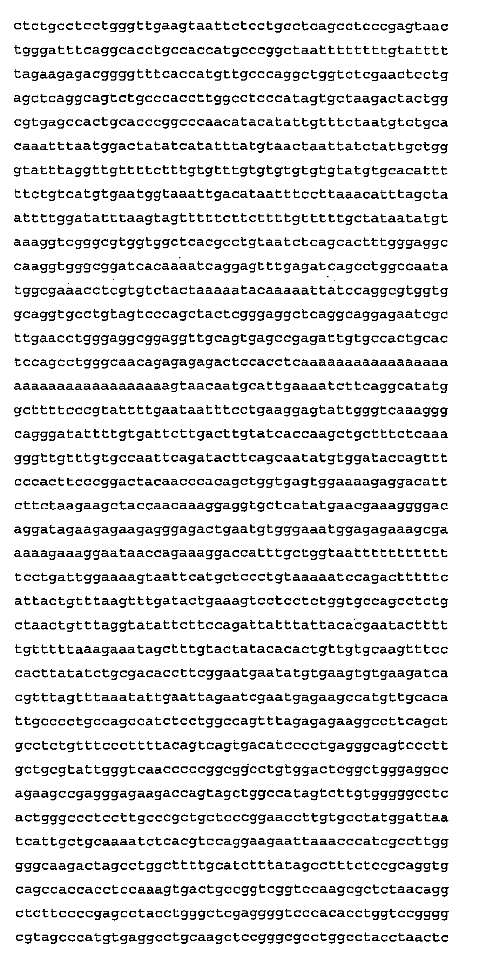

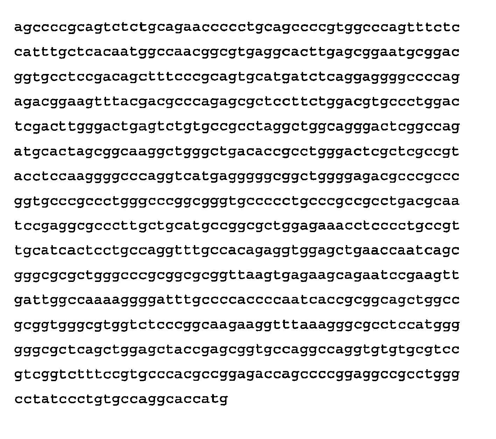

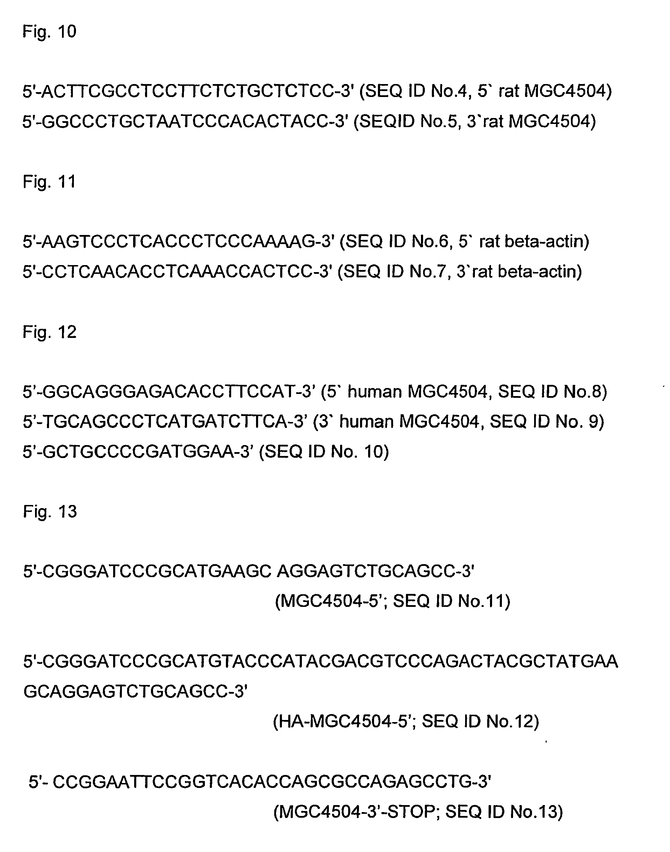

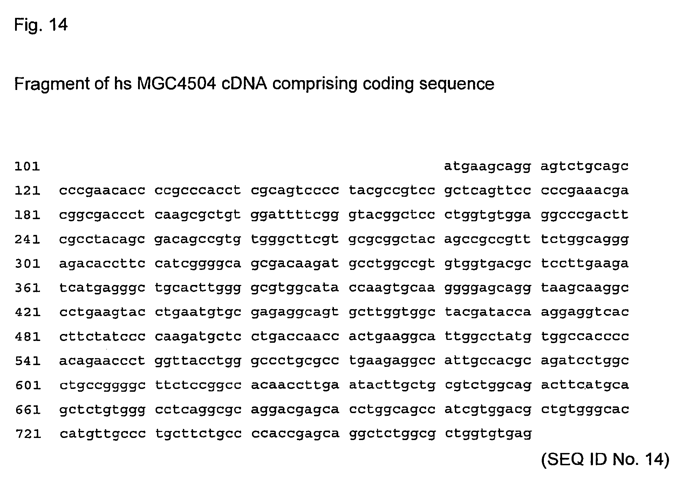

   Figure 6A: Activity of the MGC4504 5'-UTR positions -4311/+1 in different cell lines in a transient 96-well format dual-lucfferase reporter gene assay.
   Figure 6B: Experimental determination of the MGC4504 core promoter within MGC4504 5'-UTR positions -4311/+1 by assaying different truncated promoter fragments in transient 96-well format dual-luciferase reporter gene assays in HEK293 cells.
Figure 7:
   Coding Sequence of human MGC4504 (SEQ ID No.1)
Figure 8:
   Derived amino acid sequence of human MGC4504 (SEQ ID No.2)
Figure 9:
   Genomic Sequence of human MGC4504 (SEQ ID No.3)
Figure 10:
   Primers for specific amplification of rat MGC4504 mRNA (SEQ ID No.4 and 5)
Figure 11:
   Primers for specific amplification of rat beta actin mRNA (SEQ ID No. 8 and 7)
Figure 12:
   Primers for specific amplification of human MGC4504 mRNA (SEQ ID No. 8 and 9) and probe for specific hybridisation of human MGC4504 mRNA (SEQ ID No.10)
Figure 13: .
   Primers for cloning the human MGC4504 ORF from cDNA (SEQ ID No. 11 to 13)
Figure 14:
   Fragment of human MGC4504 cDNA sequence comprising the coding sequence (SEQ ID No.14)

### Example:

### Example 1:

### Gene expression of MGC4504 in an in vivo model of insulin resistance and type 2 diabetes

### Materials and Methods

### Animals and sample preparation:

Age-matched groups of obese male Zucker Diabetic Fatty (ZDF) rats (Gml, fa/fa) and their lean littermates (Gmi, +/?) were purchased from Genetic Models. The rats were housed in pairs at 20°C on a 12-h light-dark cycle with ad libitum access to water and standard rat diet (Altromin, Germany) containing 4% fat, 64% carbohydrate and 19% protein for one week upon arrival to allow recovery from transport. All experimental procedures were conducted according to the German Animal Protection Law. After 2 hrs starvation, blood samples were drawn under isofluorane anesthesia and the animals were killed by cervical disfocation. The total number of animals used for gene expression analyses was 34 animals (6 weeks old [n= 12 animals], 7 weeks old [n= 12 animals] and 12 weeks old [n= 10 animals]). Tissue probes were excised rapidly and snap-frozen in liquid nitrogen and stored at -80°C.

### Affymetrix Gene Chip^{®} analysis:

The general use of oligonucleotides arrays for gene expression monitoring has been described in US 6,177,248. In our practical application, the used micro arrays contain desoxynucleotide sequences that represent approximately 8000 known genes or EST clusters. Each gene or EST sequence is represented by up to 20 pairs of oligonucleotides, each pair consisting of one oligo that matches to a segment of the transcript, and a control oligo that contains a centrally located 1 bp mismatch. For rat, 3 arrays (RG U34A, RG U34B and RG U34C) representing approximately 24000 gene and EST sequences in total, derived from a database of known genes or EST sequences are provided by Affymetrix, Santa Clara, CA, US. 150 mg skeletal muscle probes (M. gluteus maximus) were lysed in Qiagen RLT buffer with an UtraTurrax homogenizer (Janke and Kunkel IKA Labortechnik). Total RNA from the tissue lysates was isolated with Qiagen RNeasy kit including proteinase K digestion, DNase digestion and an additional RNeasy cleanup step as recommended by the manufacturer (Qiagen). First and second strand cDNA synthesis were performed with 10µg of each total RNA using SuperScript SSII RT polymerase system (Invitrogen) and a T7(dT)₂₄ primer linking the T7 RNA polymerase promoter and oligo(deoxythymidine)₂₄. Double strand cDNA was phenol-chloroform extracted followed by ethanol precipitation and resuspended in 12µl RNAse-free water. Biotin-UPT and -CTP labelled cRNA was transcribed in vitro using Enzo BioArray High Yield RNA Transcript Labelling Kit (Enzo Diagnostics) and purified by RNeasy cleanup and ethanol precipitation. Aliquots of every total RNA and cRNA were monitored before and after each purification step by UV-spectrophotometry, agarose gel electrophoresis and BioAnalyzer RNA chips (Agilent). 15ug cRNA samples were fragmented at 94°C for 35 min in 40mM Tris / acetate pH 8.1, 100mM KOAc and 30mM MgOAc, added to hybridisation buffer and hybridised to Affymetrix GeneChips^{®} RG-U34 A, B and C micro arrays for 16 hrs at 45 °C and 60 rpm. Micro arrays were washed and double-stained with streptavidin-phycoerythrin conjugate (Molecular Probes), anti-streptavidin antibody and again streptavidin-phycoerythrin conjugate to enhance signal intensity according to the methodologies described by Affymetrix. After washing the micro arrays were analysed in a confocal GeneArray Scanner (HP) with Micro array Suite Version 4.0 software. Quality control of each chip was performed according the Affymetrix quality criteria, including mean average difference, raw intensity and 3'/5' ratio of house keeping genes beta-actin and GAPDH. Expression profiling data were analysed using an in-house software tool (GECKO 2) which performed a global normalization on all micro arrays using a reference chip for each group and the 75^{th} percentile of the median. Criteria for determining differentially expressed genes were changes in expression levels higher than 2-fold and a p-value < 0.05. Fold changes of gene expression of MGC4504 between all merged replicates of lean insulin sensitive controls and obese insulin resistant (or already diabetic, as the 12 weeks old age group) ZDF rats were analyzed using the Affymetrix qualifier RC-AI170665-AT on RGU-34 A. The results of these analyses are summarized in table 1.

In obese insulin-resistant and diabetic ZDF rats, compared to the lean insulin-sensitive controls, significantly lower levels of MGC4504 gene expression levels could be detected by Affymetrix gene chip analysis of RNA isolated from skeletal muscle biopsies. This indicates that insulin resistance and type 2 diabetes are associated with decreased gene expression levels of MGC4504.

### Quantitative real-time PCR:

1µg of total RNA isolated from each rat of the 6 and 7 weeks old groups (n= 24 animals) was reverse transcribed with AMV-RT first strand cDNA synthesis kit (Roche) in a 20µl reaction volume. 2 µl of reverse transcribed single strand cDNA was used as template for amplification in a LightCycler^{®} (Roche Diagnostics GmbH) using FastStart DNA Master Sybr Green (Roche) according to the instructions of the manufacturer. Primers used were 5' ACTTCGCCTCCTTCTCTGCTCTCC-3' (SEQ ID No.4, 5' rat MGC4504) and 5'-GGCCCTGCTAATCCCACACTACC-3' (SEQ ID No.5, 3' rat MGC4504), 5'-AAGTCCCTCACCCTCCCAAAAG-3' (SEQ ID No.6, 5' rat beta-actin) and 5'-CCTCAACACCTCAAACCACTCG3' (SEQ ID No.7, 3' rat beta-actin). The correct size of the resulting fragments (956 and 268 base pairs, respectively) was monitored by agarose gel electrophoresis. Total RNA contents were calculated using a concentration standard curve of the respective amplified fragments, normalize to expression levels of the housekeeping gene beta-actin and are summarized as fold changes of gene expression of MGC4504 between lean insulin sensitive controls and obese insulin resistant ZDF rats in Table 2.

In obese insulin-resistant and diabetic ZDF rats, compared to the lean insulin-sensitive controls, significantly lower levels of MGC4504 gene expression levels could be detected by quantitative real-time PCR analysis of RNA isolated from skeletal muscle biopsies. This indicates that insulin resistance and type 2 diabetes are associated with decreased gene expression levels of MGC4504.

### Example 2:

### Gene expression of MGC4504 in human tissues

### Materials and Methods

### TaqMan analysis of MGC4504 gene expression in human tissues:

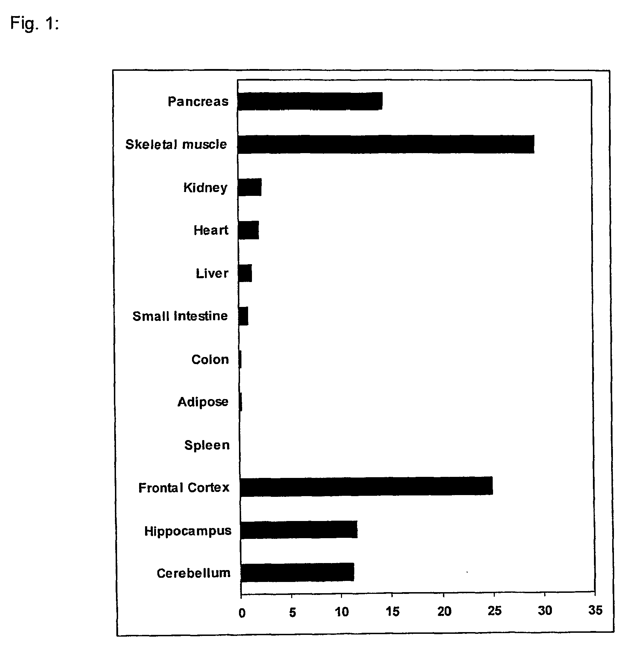

MGC4504 expression levels in various human tissues were determined by TaqMan analysis using primers 5'-GGCAGGGAGACACCTTCCAT-3' (5' human MGC4504, SEQ ID No.8) and 5'-TGCAGCCCTCATGATCTTCA-3' (3' human MGC4504, SEQ ID No. 9) and probe 5'-GCTGCCCCGATGGAA-3' (SEQ ID No. 10). All values were normalized for human beta-2-microglobulin expression levels to standardize for equal loading. Figure 1 summarizes MGC4504 gene expression levels in a selected subset of different human tissues tested.

TaqMan analysis revealed that highest MGC4504 expression is detected in different parts of the brain, skeletal muscle, pancreas and kidney. This indicates that MGC4504 expression is almost restricted to muscle and pancreas.

### Example 3:

### Effects of MGC45045 overexpression on glucose metabolism and insulin secretion

### Materials and Methods

### Cloning and plasmids:

The complete ORF of human MGC4504 was amplified from human skeletal muscle cDNA by PCR using 5'-primers MGC4504-5': 5'-CGGGATCCCGCATGAAGC AGGAGTCTGCAGCC-3' (SEQ ID No.11) or HA-MGC4504-5': 5'-CGGGATCCC GCATGTACCCATACGACGTCCCAGACTACGCTATGAAGCAGGAGTCTGCAGCC-3' (SEQ ID No.12) and MGC4504-3'-STOP: 5'- CCGGAATTCCGGTCAC ACCAGCGCCAGAGCCTG-3' (SEQ ID No.13). The latter 5' primer introduced a sequence encoding for an amino-terminal in-frame HA-epitope tag. The resulting fragments were cloned into pGEX-6P1 and pcDNA3.1 (+) hygro vector (Invitrogen) via BamHI / EcoRI or via BamHI (5') and blunt-ended (3') EcoRI (insert) and NotI (vector) sites to obtain pGEX-6P1 MGC4504 or pcDNA3.1(+) hygro HA-MGC4054, respectively. The identities of all constructs were confirmed by sequencing.

### Cell culture, transfections and immunofluorescence:

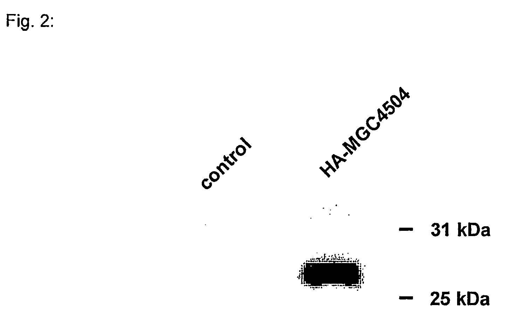

Rat L6GLUT4myc myoblasts expressing myc-epitope tagged GLUT4 were a kind gift of A. Klip (The Hospital for Sick Children, Toronto, Ontario, Canada). L6 (ATCC#: CRL-1458) and L6GLUT4myc were maintained in alpha-MEM with Glutamax (Gibco #32571) supplemented with 10% FCS gold (PAA, A 15-609), penicillin/streptomycin solution (PAA #P11-010). L6GLUT4myc medium was additionally supplemented with 2µg/ml blasticidin (Calbiochem #203350) to select for GLUT4myc expression. RIN-5F rat insulinoma cells (ATCC#: CRL-2058) were maintained in RPMI 1640 with HEPES/L-glutamine/NaHCO₃ (GIBCO # 52400-025) supplemented with 10% FCS gold, (PAA, #A15-609), penicillin/streptomycin solution (PAA #P11-010) and 1mM sodium pyruvate (Gibco #11360-039). HEK293 cells (ATCC#: CRL-1573) were maintained in DMEM (GIBCO # 41965-039) supplemented with 10% FCS gold (PAA, A 15-609), penicillin/streptomycin solution (PAA #P11-010) and 1mM L-glutamine (Gibco #25030-032). All cell lines were cultured at 37°C in 5% CO₂ and 95% humidity and were subcultured twice weekly. Transfections for studying protein expression were carried out with L6GLUT4myc or RIN-5F cells grown in 6-well plates to 60 % confluency, 2.5 µg plasmid DNA and Fugene 6 reagent (Roche) as recommended by the manufacturer. In all transient transfections or stably selected cell clones, empty pcDNA3.1(+) hygro vector served as negative control (refered to as WT cells). Stable cell clones were selected with 500µg/ml hygromycin B, resistant single cell clones were picked manually and expanded. HA-MGC4504 expression levels were determined by SDS-PAGE and Western blotting (NuPAGE, Invitrogen) using anti-HA 3F10 monoclonal (Roche) and anti-rat horseradish peroxidase secondary antibodies and ECL chemoluminiscent detection (Amersham). Immunofluorescence assays were performed as described (Voss et al, 2001) using stable L6GLUT4myc or RIN-5F HA-MGC4504 cells or L6GLUT4myc or RIN-5F cells transiently transfected with pcDNA3.1(+) HA-MGC4504 grown on cover slips to 70 % confluency. HA-MGC4504 expression was monitored using anti-HA 3F10 antibody (Roche) and anti-rat alexa fluor 488 antibody (Molecular Probes). Nuclei were visualized using 1µM ToPro iodide in PBS (Molecular Probes). Confocal images were taken with a Leica TCS SP2 confocal laser scanning microscope. Figure 2 shows the stable expression of HA-MGC4504 in L6GLUT4myc myoblasts. Figure 3 shows the sub cellular distribution of MGC4504 in cytoplasm and nucleus of transiently transfected L6GLUT4myc (Figure 3A) and RIN-5F (Figure 3B) cells.

MGC4504 cDNA can be readily transfected and HA epitope-tagged MGC4504 protein be expressed in rat myoblast and insulinoma cells where it is localized in cytoplasm and prominent in the nucleus.

### 2-Deoxyglucose uptake and analysis of insulin secretion:

L6GLUT4myc cells or L6GLUT4myc HA-MGC4504 and WT cell clones were plated in 96-well Cytostar-T™ scintillating micro plates (Amersham) at 4.0x10⁴ viable cells per well. After 32 hrs, the cells were serum-starved with alpha-MEM supplemented with 2% new-born calf serum (PAA) and penicillin/streptomycin solution (PAA #P11-010) for 16 hrs. For analyzing glucose uptake, the cells were washed twice in Krebs-Ringer Buffer pH 7.3 (KRB) and incubated for 25 min with the given insulin concentrations in KRB. ¹⁴C 2-deoxyglucose (0.3 µCi per well) was added and the cells were incubated for another 25 min in a total volume of 150 µl per well. The uptake was stopped by adding 40µM cytochalasin B, sealed and scintillation was measured in a Wallac micro beta counter (Perkin Elmer). Unspecific uptake was determined by incubating control wells with 20µM cytochalasin B and subtracted from each value. For monitoring insulin secretion. RIN-5F cells or RIN-5F HA-MGC4504 and WT cell clones were plated in 12-well plates and grown for 24 hrs. After serum-starvation with alpha-MEM supplemented with 2% new-born calf serum (PAA) and antibiotics (Pen/Strep) for 16 hrs, the cells were washed twice in KRB and incubated for 2hrs in KRB with different amounts of glucose (0-20 mM). The insulin content of the supernatants was determined by using a Rat Insulin ELISA (Mercodia) according to the manufacturers protocol and the cells were further lysed for determination of the protein content to ensure comparable loading. Figure 4A shows a typical 2-deoxyglucose uptake assay and increased insulin sensitivity of L6GLUT4myc HA-MCG4504 cells. Figure 4B a typical insulin secretion assay and increased basal and glucose stimulated insulin secretion of RIN-5F HA-MGC4504 cells. These results show that MGC4504 expression Is linked to Insulin sensitivity and glucose disposal as well as insulin secretion, since overexpression of MGC4504 leads to increased insulin sensitivity and increased insulin-stimulated glucose-uptake in a muscle cell line as well as increased glucose-stimulated Insulin secretion in an insulinoma cell line.

### Example 4:

### Recombinant expression of soluble recombinant MGC4504 fusion protein

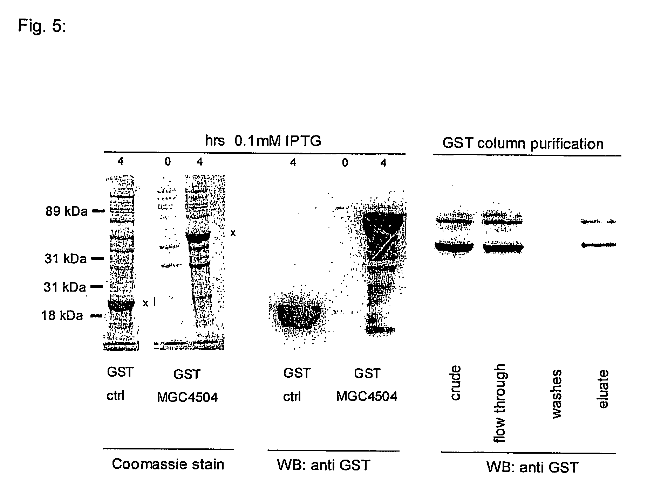

E. coli strain BL21DE3 bacteria (Invitrogen) were transformed with pGEX-6P1 empty vector or pGEX-6P1 MGC4504 and grown in LB, Induced with 0.1 mM IPTG at a OD₆₀₀ of 0.6 and grown for 4 hrs. Cells were lysed and purified by GST- affinity chromatography according to the GST-micro spin column standard protocol from Amersham and expression of GST and GST-MGC4504 proteins monitored by SDS-PAGE and western blotting using anti-GST antibody (Amersham). Figure 5 shows different expression and purification steps of soluble GST-MGC4504 protein, indicating that recombinant soluble MGC4504 protein can readily be obtained in an E. coli expression system.

### Example 5:

### HT5-amenable assay for determination of compounds modulating MGC4504 expression

### Materials and methods:

The 5'-UTR of human MGC4504 comprising nucleotides -4311 to +1 (relative to the transcriptional start: (nts 84361 - 88681 in genomic sequence AC020661) was PCR amplified and cloned via Xhol and BgIII into pGL3neo basic resulting in pGL3neo basic MGC4504 -4311/+1. Further truncated constructs were generated by digesting pGL3neo basic MGC4504 -4311/+1 with BfrBl, Xhol or Avril, subsequent klenow-blunting and BgIII digestion. The resulting fragments were ligated to KpnI-digested, Klenow-blunted and subsequently BgIII-digested pGL3neo basic vector to obtain pGL3neo basic MGC4504 -19441+1, -847/+1 and -546/+1 plasmids. The identities of all constructs were confirmed by sequencing RIN-5F rat insulinoma cells were maintained in RPMI 1640 with HEPES/L-glutamine/NaHCO₃ (GIBCO # 52400-025) supplemented with 10% FCS gold, (PAA, #A15-609), penicillin/streptomycin solution (PAA #P11-010) and 1mM sodium pyruvate (Gibco #11360-039). HEK293 cells were maintained in DMEM (GIBCO # 41965-039) supplemented with 10% FCS gold (PAA, A 15-609), penicillin/streptomycin solution (PAA #P11-010) and 1mM L-glutamine (Gibco #25030-032). All cell lines were cultured at 37°C in 5% CO₂ and 95% humidity and were subcultured twice weekly. RIN-5F cells were transiently transfected with 1 ng pCMV-RL (Promega) and 0.4ng pBluescript-SK+ (Stratagene) as carrier and different amounts of pGL3.1neo basic empty vector or promoter constructs pGL3.1neo basic MGC4504-5'-UTR-4311/+1, -1944/+1,-8471+1 or-5461+1 by using Fugene6 (Roche) in 96-well microtiter plates (Coming Costar #3610). HEK cells were transfected by using Polyfect (Qiagen). Transfections were carried out according to the manufacturers protocol. Concentrations of the pGL3.1 neo basic constructs were optimized to 10ng (HEK) and 40ng (RIN5F) plasmid per well to obtain maximal reporter activity. All plasmids used for transfections were isolated using Qiagen Endofree MaxiPrep kits according to the manufacturers protocol. After 48hrs, the cells were lysed, firefly and renilla luciferase activities determined according to the manufacturers protocol (Promega Dual-luciferase System) by measuring the respective activities in a Wallac microbeta counter (Perkin Elmer), Firefly luciferase values were normalized for renilla luciferase activities and plotted as fold induction over empty pGL3.1neo basic controls. A stable HEK293 cell line was generated with pGL3.1 neo basic MGC4504-5'-UTR - 4311/+1 and selected with 500 µg/ml geneticin (Gibco) and positive clones selected for luciferase activities. Resulting HEK MGC4504 5'-UTR -4311/+1 cells were plated in 96-well microtiter plates (Corning Costar #3610) and grown for 24 hrs. Compounds were dissolved to a concentration of 10mM in DMSO and diluted to the respective working concentrations (10 µM to 100 pM) in DMEM medium (#41965-039, Invitrogen) supplemented with 2 % Ultroser (#12039-012, Biosepra), 1 % Penicillin-Streptomycin solution (#15140-122, Invitrogen) and 2 mM L-Glutamine (#25030-024, Invitrogen). To assay for compounds able to modulate the promotor/transcriptional activity of the MGC4504 promoter constructs, medium can be aspirated from the cells and e.g.100µl of the medium containing the respective diluted compounds can be added directly. After e.g. 48 hrs incubation, firefly luciferase activities can be determined as described above. Raw data can be transferred into excel-files and the ratio of firefly/renilla activities determined for each well. Dose/activity relationships can be determined in XL-Fit according to the manufacturers protocol (IDBS). Figure 6 shows the activity of the MGC4504 5'-UTR positions -4311/+1 in different cell lines in a transient 96-well format dual-luciferase reporter gene assay. These results show that MGC4504 promoter activity can be assayed In a HTS-amenable reporter-gene assay giving a sufficient signal-to-noise ratios in cell lines reflecting the naturally occuring expression pattern of MGC4504.

Furthermore, the minimal promoter necessary for MGC4504 is comprised within MGC4504 5'-UTR position -5461+1.

### Example 6:

### Hypothetical, HTS-amenable cellular assay testing a substance for its ability to positively influence/stimulate MGC4504 protein activity

To assay for MGC4504 protein activity, one of the methods according to present invention could be performed utilizing a cell only weakly expressing (endogenously or heterologously) MGC4504 and having weak or moderate Insulin sensitivity, contacting the cell with a test substance and testing whether the insulin sensitivity is modulated (i.e. increased or decreased). The employment of one or more negative control cells ensures, that the substance does not modulate insulin sensitivity by modulating the MGC4504 amount present in said cell (on transcript or protein level) but by modulating MGC4504 protein activity.

A cell line (e.g. HEK293) is stably transfected with an expression vector comprising MGC4504 according to SEQ ID No.1 under the control of a promoter like SV40 as in pCDNA3.1 + hygro HA-MGC4504 (or a weaker promotor) according to standard procedures. As a control, , the same cell line (e.g. HEK293) is stably transfected with the same expression vector comprising a reporter gene like renilla luciferase under the control of the same promoter (e.g. pCDNA3.1 luciferase). After Incubation of both cells with the test substance, the Insulin-stimulated glucose uptake is determined for both cells according to standard procedures in presence and In absence of the test substance. For the control cell, luciferase activity is also determined by standard procedures in presence and in absence of the test substance. Those substances that are able to increase insulin-stimulated glucose uptakein the cell expressing MGC4504 and do not increase luciferase activity or the insulin sensitivity of the control cells not expressing MGC4504 are substances that can be considered to increase MGC4504 activity.

### Example 7

### Gene expression of MGC4504 in a diet-Induced model of type 2 diabetes

### Materials and Methods

### Animals and sample preparation:

Age-matched female Zucker Diabetic Fatty (ZDF) rats (Gmi, fa/fa) were purchased from Genetic Models. At a time three rats were housed at 20°C on a 12-h light-dark cycle with ad libitum access to water and a high fat diet diet (Research Diets, USA) for several weeks. All experimental procedures were conducted according to the German Animal Protection Law. The high fat diet fed animals could be divided in two subgroups: the non-responder group did not develop a type 2 diabetes state (blood glucose « 12 mM), whereas the responder group did (blood glucose > 12 mM). Seven animals of the responder group and six animals of the non-responder group were anesthetized with isofluorane and killed by cervical dislocation. Pancreas samples were excised rapidly and transferred to RNAlater, long-term storage was carried out at -80°C.

Affymetrix Gene Chip^{®} analysis and RNA isolation was performed as described in example 1 with two exceptions: no proteinase K digestion was performed while RNA preparation and hybridization was carried out using RAE 230_2 arrays representing about 28000 rat genes. For hybridization, the isolated RNAs were combined at a time to two pools for each group (non-responder and responder). Data analysis was performed using Resolver 4.0 (Rosetta Inc, USA) according to the software producer instructions.

Criteria for determining differentially expressed genes were changes in expression levels higher than 1.5-fold and a p-value < 0.001. Fold change of gene expression of MGC4504 between non-responder and responder groups was analyzed using the Affymetrix qualifier 1389573_at on RAE 230_2. The result of this analysis is summarized in Table 3.

In the responder group developing type 2 diabetes, compared to the non-responding animals, significantly lower levels of MGC4504 gene expression levels could be detected by Affymetrix Gene Chip^{®} analysis of RNA isolated from pancreas biopsies. This indicates that development of type 2 diabetes, caused by a high fat diet, is associated with decreased gene expression levels of MGC4504.

### Literature

Voss, M.D., Hille, A., Barth, S., Spurk, A., Hennrich, F., Holzer, D., Mueller-Lantzsch, N., Kremmer, E., Grasser, F.A. (2001) J. Virol 75, 11781-90
Literature for standard laboratory methods
If not indicated otherwise, standard laboratory methods were or can be performed according to procedures disclosed in the following standard literature:
Sambrook et al. (1989) Molecular Cloning: A Laboratory Manual. Second edition. Cold Spring Harbor Laboratory Press, Cold Spring Harbor, NY. 545 pp;
Current Protocols in Molecular Biology; regularly updated, e.g. Volume 2000; Wiley & Sons, Inc; Editors: Fred M. Ausubel, Roger Brent, Robert Eg. Kingston, David D. Moore, J.G. Seidman, John A. Smith, Kevin Struhl.
Current Protocols in Human Genetics; regularly uptdated; Wiley & Sons, Inc; Editors: Nicholas C. Dracopoli, Honathan L. Haines, Bruce R. Korf, Cynthia C. Morton, Christine E. Seidman, J.G. Seigman, Douglas R. Smith.
Current Protocols in Protein Science; regularly updated; Wiley & Sons, Inc; Editors: John E. Coligan, Ben M. Dunn, Hidde L. Ploegh, David W. Speicher, Paul T. Wingfield.
Molecular Biology of the Cell; third edition; Alberts, B., Bray, D., Lewis, J., Raff, M., Roberts, K., Watson, J.D.; Garland Publishing, Inc. New York & London, 1994;
Short Protocols in Molecular Biology, 5th edition, by Frederick M. Ansubel (Editor), Roger Brent (Editor), Robert E. Kingston (Editor), David D. Moore (Editor), J.G. Seidman (Editor), John A. Smith (Editor), Kevin Struhl (Editor), October 2002, John Wiley & Sons, Inc., New York"
Transgenic Animal Technology: A Laboratory Handboook. C.A. Pinkert, editor; Academic Press Inc., San Diego, California, 1994 (ISBN: 0125571658)
Gene targeting: A Practical Approach, 2nd Ed., Joyner AL, ed. 2000. IRL Press at Oxford University Press, New York;
Manipulating the Mouse Embryo: A Laboratory Manual. Nagy, A, Gertsenstein, M., Vintersten, K., Behringer, R., 2003, Cold Spring Harbor Press, New York;

**Table 1:**

| **ZDF rats 6 weeks old** | | **ZDF rats 7 weeks old** | | **ZDF rats 12 weeks old** | |
|---|---|---|---|---|---|
| **Fold change** | **P-value** | **Fold change** | **P-value** | **Fold change** | **P-value** |
| - 3.92 | 0.0053 | - 6.18 | 0.0008 | -4.87 | 0.0005 |

**Table2:**

| **ZDF rats 6 weeks old** | | **ZDF rats 7 weeks old** | |
|---|---|---|---|
| **Fold change** | **P-value** | **Fold change** | **P-value** |
| - 2.98 | 0.0047 | - 3.32 | 0.0076 |

**Table 3:**

| **Female ZDF rats with diet-induced diabetes, pancreas** | |
|---|---|
| **Fold change** | **P-value** |
| -1.77 | 1.29E-06 |

## Claims

1. The use of MGC4504 protein, a functional derivative or fragment thereof, or a nucleic acid coding for said protein, derivative or fragment thereof, for the identification of substances active in preventing or treating metabolic syndrome, obesity, dislipidemia, diabetes mellitus type I or II or insulin resistance and preferably insulin resistance, wherein the function of the MGC4504- protein fragment is defined as the capability of improving/increasing insulin release from pancreatic beta cells, improving/increasing insulin-stimulated glucose disposal, influencing insulin signalling or insulin action or increasing the insulin-stimulated glucose uptake and wherein the derivative of the nucleic acid comprises a phosphorothioate modification and wherein the derivative of the protein or fragment thereof is the protein or fragment thereof coupled to a cell-permeant phosphopeptide.

2. A method for identifying substances active in preventing or treating metabolic syndrome, obesity, dislipidemia, diabetes mellitus type I or II or insulin resistance and preferably insulin resistance comprising:
a Contacting a cell, which has a decreased activity or amount of MGC4504, with a test substance;
b. Determining whether the substance is able to increase the activity or amount of MGC4504 present in the cell.

3. A method for identifying substances active in preventing or treating metabolic syndrome, obesity, dislipidemia, diabetes mellitus type I or II or insulin resistance and preferably insulin resistance comprising:
a. Providing a cell transfected with a nucleic acid vector comprising the MGC4505 promoter or a functional fragment thereof operationally coupled to a reporter gene or a functional fragment thereof;
b. Providing a cell transfected with a control vector which comprises a reporter gene or a functional fragment thereof not being operationally coupled to a functional MGC4504 promoter;
c. Determining the reporter gene activity of the cell according to a) and b) in the presence of a test substance;
d. Determining the reporter gene activity in the absence of said substance, wherein an active substance is a substance capable of increasing reporter gene activity according to a) without increasing reporter gene activity of b).

4. Use of a means for the detection of MGC4504 for diagnosing metabolic syndrome, obesity, dislipidemia, diabetes mellitus type I or II or insulin resistance and preferably insulin resistance or a predisposition for metabolic syndrome, obesity, dislipidemia, diabetes mellitus type I or II or insulin resistance and preferably insulin resistance in an isolated sample of an individual, thereby using a means to detect MGC4504 mRNA which is a mRNA probe or a primer set able to amplify MGC4504 mRNA or cDNA, or using a means to detect MGC4504 protein which is a specific MGC4504 antibody, or using a means to detect mutations in the MGC4504 nucleic acid which is a nucleic acid probe or a primer set able to amplify MGC4504 nucleic acid, or using a means to detect mutations in the MGC4504 protein which is a specific antibody or using a means to specifically detect MGC4504 genomic DNA which comprises or is a specific MGC4504 nucleic acid probe or a primer set capable of amplifying MGC4504 genomic or cDNA or a fragment thereof.

5. The use of claim 4, wherein the means is a primer consisting of the nucleotide sequence according to SEQ ID NO: 8 or with a nucleotide sequence according to SEQ ID NO: 9, or a nucleic acid probe consisting of the nucleotide sequence according to SEQ ID NO: 10.

6. Method of diagnosing metabolic syndrome, obesity, dislipidemia, diabetes mellitus type I or II or insulin resistance and preferably insulin resistance or a predisposition for metabolic syndrome, obesity, dislipidemia, diabetes mellitus type I or II or insulin resistance and preferably insulin resistance comprising analyzing the amount of MGC4504 present in an isolated sample of an individual, wherein a lowered MGC4504 amount present in said sample in comparison to one or more reference samples is indicative of the predisposition or disease, and wherein the reference sample is an isolated sample having an average activity or amount of MGC4504 and/or a sample of a donor not having a disease or a predisposition for a disease associated with or caused by a malfunction of the carbohydrate or lipid metabolism.

7. Method of diagnosing metabolic syndrome, obesity, dislipidemia, diabetes mellitus type I or II or insulin resistance and preferably insulin resistance or a predisposition for metabolic syndrome, obesity, dislipidemia, diabetes mellitus type I or II or insulin resistance and preferably insulin resistance comprising analyzing an isolated sample of an individual for mutations of MGC4504 in comparison to a reference sequence of MGC4504, wherein the presence of a mutation is indicative of the disease or the predisposition.

8. Method of diagnosing metabolic syndrome, obesity, dislipidemia, diabetes mellitus type I or II or insulin resistance and preferably insulin resistance or a predisposition for metabolic syndrome, obesity, dislipidemia, diabetes mellitus type I or II or insulin resistance and preferably insulin resistance comprising analysing an isolated sample of an individual for an impaired or lowered MGC4504 activity in comparison to a reference sample, wherein the presence of an impaired or a lowered activity is indicative of the disease or the predisposition, and wherein the reference sample is an isolated sample having an average activity or amount of MGC4504 and/or a sample of a donor not having a disease or a predisposition for a disease associated with or caused by a malfunction of the carbohydrate or lipid metabolism.

9. Kit for the identification of metabolic syndrome, obesity, dislipidemia, diabetes mellitus type I or II or insulin resistance and preferably insulin resistance or a predisposition for metabolic syndrome, obesity, dislipidemia, diabetes mellitus type I or II or insulin resistance and preferably insulin resistance comprising at least one means for the detection of MGC4504, wherein the means is a primer consisting of the nucleotide sequence according to SEQ ID NO. 8 or with a nucleotide sequence according to SEQ ID NO. 9 or a nucleic acid probe consisting of the nucleotide sequence according to SEQ ID NO. 10.

10. MGC4504 or a functional fragment or derivative thereof or a composition of matter comprising MGC4504 or a functional fragment or derivative thereof for use in a method for the treatment or prevention of metabolic syndrome, obesity, dislipidemia, diabetes mellitus type I or II or insulin resistance and preferably insulin resistance, wherein the function of the MGC4504 protein fragment is defined as the capability of improving/increasing insulin release from pancreatic beta cells, improving/increasing insulin-stimulated glucose disposal, influencing insulin signalling or insulin action or increasing the insulin-stimulated glucose uptake and wherein the derivative of the MGC4504 nucleic acid comprises a phosphorothioate modification and wherein the derivative of the MGC4504 protein or fragment thereof is the protein or fragment thereof coupled to a cell-permeant phosphopeptide.

11. In vitro-use of a cell heterologously expressing MGC4504 for the identification of substances active in the treatment or prevention of metabolic syndrome, obesity, dislipidemia, diabetes mellitus type I or II or insulin resistance and preferably insulin resistance.

12. Use of a non-human animal heterologously expressing MGC4504 for the identification of substances active in the treatment or prevention of metabolic syndrome, obesity, dislipidemia, diabetes mellitus type I or II or insulin resistance and preferably insulin resistance.

13. In vitro-use of a MGC4504 knockout cell for the identification of substances active in the treatment or prevention of metabolic syndrome, obesity, dislipidemia, diabetes mellitus type I or II or insulin resistance and preferably insulin resistance.

14. Use of a non-human MGC4504 knockout animal for the identification of substances active in the treatment or prevention of metabolic syndrome, obesity, dislipidemia, diabetes mellitus type I or II or insulin resistance and preferably insulin resistance.

15. Primer consisting of the nucleotide sequence according to SEQ ID No. 8 or with a nucleotide sequence according to SEQ ID NO. 9.

16. Nucleic acid probe consisting of the nucleotide sequence according to SEQ ID No. 10.

17. High throughput screen (HTS) comprising a method according to claim 2 or 3.

18. Use according to claim 1, MGC4504 or a functional fragment thereof or a composition of matter comprising MGC4504 or a functional fragment thereof according to claim 10, method according to claim 2 or HTS according to claim 17, wherein MGC4504, the derivative or fragment thereof is used as an isolated molecule.

19. Use according to claim 1, method according to claim 2, or HTS according to claim 17, wherein MGC4504, the derivative or fragment thereof is used in a biochemical or cellular assay.

20. Use, method or HTS according to claim 19, wherein the assay is a cellular assay and the cells are transiently or stably and preferably stably transfected to heterologously express MGC4504.

21. Use, method or HTS according to claim 20 or use according to claim 11 or 12, wherein the cell is a mammalian cell.

22. Use, method or HTS according to claim 21, wherein the cell is a rodent cell and preferably a murine cell.

23. Use, method or HTS according to claim 21, wherein the cell is a human cell.

24. Use according to claim 12 or 14, wherein the animal is a vertebrate or an invertebrate, preferably a vertebrate, more preferably a non-human mammal and even more preferably a mouse or a rat.

25. Use according to claim 11 or 13, wherein the heterologously expressed MGC4504 is a transgene.

26. Use according to claim 1, MGC4504 or a functional fragment thereof or a composition of matter comprising MGC4504 or a functional fragment thereof according to claim 10, method according to claim 2 or 3 or HTS according to claim 17, wherein MGC4504 is mammalian and preferably human MGC4504.

27. Use according to claim 4 or method according to one of the claims 6 to 8, wherein the individual is a human individual.

28. Use according to claim 1, MGC4504 or a functional fragment thereof or a composition of matter comprising MGC4504 or a functional fragment thereof according to claim 10, method according to one of the claims 2, 6 or 7, kit according to claim 9, or HTS according to claim 17, wherein MGC4504 or the derivative or fragment thereof is a nucleic acid.

29. Use, method or high throughput screen according to claim 28 or method according to claim 3, wherein the nucleic acid comprises or consists of one of the following sequences:
a. The sequence according to SEQ ID No. 1 or 14;
b. A sequence capable of hybridizing with a sequence according to a) at conditions of high stringency and coding for a protein or polypeptide with MGC4504 function;
c. A sequence derived from a sequence according to a) or b) due to the degeneracy of the genetic code and coding for a protein or polypeptide with MGC4504 function;
d. A fragment of one of the sequences according to a), b) or c), coding for a polypeptide with MGC4504 function, wherein the protein or polypeptide has a polypeptide sequence comprising or consisting of the sequence according to SEQ ID No. 2;
wherein the MGC4504 function according to b. to d. is defined as the capability of improving/increasing insulin release from pancreatic beta cells, improving/increasing insulin-stimulated glucose disposal, influencing insulin signalling or insulin action or increasing the insulin-stimulated glucose uptake.

30. Use according to claim 1, MGC4504 or a functional fragment thereof or a composition of matter comprising MGC4504 or a functional fragment thereof according to claim 10, method according to one of the claims 2, 6, or 7, kit according to claim 9 or high throughput screen according to claim 17, wherein MGC4504 or the derivative or fragment thereof is a protein or polypeptide.

31. Use, method, kit or high throughput screen according to claim 30, use according to claim 1 or method according to claim 2, wherein the polypeptide or protein is encoded by one of the following sequences:
a. The sequence according to SEQ ID No. 1 or 14;
b. A sequence capable of hybridizing with the sequence according to a) at conditions of high stringency and which is coding for a protein or polypeptide with MGC4504 function;
c. A sequence derived from a sequence according to a) or b) due to the degeneracy of the genetic code and coding for a protein or polypeptide with MGC4504 function;
d. A fragment of one of the sequences according to a), b) or c), coding for a protein or polypeptide with MGC4504 function;
wherein the MGC4504 function according to b. to d. is defined as the capability of improving/increasing insulin release from pancreatic beta cells, improving/increasing insulin-stimulated glucose disposal, influencing insulin signalling or insulin action or increasing the insulin-stimulated glucose uptake.

32. Use, method, kit, high throughput screen according to claim 30 or 31, wherein the protein or polypeptide comprises or consists of the sequence according to SEQ ID No.2.

33. Use, method or high throughput screen according to one of the previous claims, wherein the modulation is an activation.

34. Method according to claim 3, wherein the reporter gene is a gene encoding renilla or firefly luciferase, green or blue fluorescent protein, beta-galactosidase, or chloramphenicol-acetyl-transferase.

35. Method or use according to one of the claims 2, 3, 11 or 12, wherein the cell is a primary cell or belongs to a cell line.

36. Method or use according to claim 35, wherein the cell is a HEK293, RIN-5F, CHO or NTH3T3cell.

37. Use according to claim 11, wherein the cell is isolated from a non-human transgenic animal.

38. Use according to claim 13, wherein the cell is isolated from a non-human knockout animal.

39. Method according to claim 3, wherein the transfection is a transient or stable transfection and preferably a stable transfection.

40. Method of treatment according to claim 8 or 27 comprising endogenously increasing the MGC4504 steady state level.

41. Method according to claim 6 comprising the use of a means to specifically analyze the MGC4504 mRNA level which is a specific MGC4504 nucleic acid probe or a primer set capable of specifically amplifying MGC4504 cDNA or a fragment thereof, wherein the function of the MCG protein encoded by the fragment is defined as the capability of improving/increasing insulin release from pancreatic beta cells, improving/increasing insulin-stimulated glucose disposal, influencing insulin signaling or insulin action or increasing the insulin-stimulated glucose uptake.

42. Method according to claim 6 comprising the use of a means to specifically analyze the MGC4504 protein level which is a specific MGC4504 antibody.

43. The method according to claim 6 using a means to detect MGC4504 mRNA which is a mRNA probe or a primer set able to amplify MGC4504 mRNA or cDNA.

44. The method according to claim 6 using a means to detect MGC4504 protein which is specific MGC4504 antibody.

45. The method according to claim 7 using a means to detect mutations in the MGC4504 nucleic acid which is a nucleic acid probe or a primer set able to amplify MGC4504 nucleic acid.

46. The method according to claim 7 using a means to detect mutations in the MGC4504 protein which is a specific antibody.

47. Use of a knock out cell or animal according to claim 13 or 14, wherein the knock out is a functional or a genomic knock out.

## Patentansprüche

1. Verwendung von MGC4504-Protein, einem funktionellen Derivat oder Fragment davon oder einer Nukleinsäure, die für das Protein kodiert, eines Derivats oder Fragments davon zur Identifizierung von Substanzen, die zur Vorbeugung oder Behandlung von metabolischem Syndrom, Fettleibigkeit, Dyslipidämie, Diabetes mellitus Typ I oder II oder Insulinresistenz und vorzugsweise Insulinresistenz wirksam sind, wobei die Funktion des MGC4504-Proteinfragments definiert ist als die Fähigkeit, die Insulinfreisetzung aus den beta-Zellen des Pankreas zu verbessern/zu erhöhen, die Insulin-stimulierte Glucose-Lagerung zu verbessern/zu erhöhen, die Insulin-Signalgebung oder die Insulinwirkung zu beeinflussen oder die Insulin-stimulierte Glucose-Aufnahme zu erhöhen, und wobei das Derivat der Nukleinsäure eine Phosphorthioat-Modifikation umfasst und wobei es sich bei dem Derivat des Proteins oder dem Fragment davon um das Protein oder das Fragment davon, gekoppelt an ein zellpermeierendes Phosphopeptid, handelt.

2. Verfahren zur Identifizierung von Substanzen, die zur Vorbeugung oder Behandlung von metabolischem Syndrom, Fettleibigkeit, Dyslipidämie, Diabetes mellitus Typ I oder II oder Insulinresistenz und vorzugsweise Insulinresistenz wirksam sind, bei dem man:
a. eine Zelle mit einer verringerten Aktivität oder Menge von MGC4504 mit einer Testsubstanz in Kontakt bringt,
b. bestimmt, ob die Substanz in der Lage ist, die Aktivität oder Menge des in der Zelle vorhandenen MGC4504 zu erhöhen.

3. Verfahren zur Identifizierung von Substanzen, die zur Vorbeugung oder Behandlung von metabolischem Syndrom, Fettleibigkeit, Dyslipidämie, Diabetes mellitus Typ I oder II oder Insulinresistenz und vorzugsweise Insulinresistenz wirksam sind, bei dem man:
a. eine Zelle bereitstellt, die mit einem Nukleinsäurevektor transfiziert ist, der den MGC4504-Promotor oder ein funktionelles Fragment davon umfasst, das funktionsfähig mit einem Reportergen oder einem funktionellen Fragment davon gekoppelt ist,
b. eine Zelle bereitstellt, die mit einem Kontrollvektor transfiziert ist, der ein Reportergen oder ein funktionelles Fragment davon umfasst, das nicht funktionsfähig mit einem funktionellen MGC4504-Promotor gekoppelt ist,
c. die Reportergen-Aktivität der Zelle gemäß a) und b) in Gegenwart einer Testsubstanz bestimmt,
d. die Reportergen-Aktivität in Abwesenheit der Substanz bestimmt, wobei eine Wirksubstanz eine Substanz ist, die die die Reportergen-Aktivität gemäß a) erhöhen kann, ohne die Reportergen-Aktivität gemäß b) zu erhöhen.

4. Verwendung eines Mittels zum Nachweis von MGC4504 zur Diagnose von metabolischem Syndrom, Fettleibigkeit, Dyslipidämie, Diabetes mellitus Typ I oder II oder Insulinresistenz und vorzugsweise Insulinresistenz oder einer Prädisposition für metabolisches Syndrom, Fettleibigkeit, Dyslipidämie, Diabetes mellitus Typ I oder II oder Insulinresistenz und vorzugsweise Insulinresistenz in einer isolierten Probe von einem Individuum, wobei man ein Mittel zum Nachweis von MGC4504-mRNA, bei dem es sich um eine mRNA-Sonde oder einen Primersatz handelt, der MGC4504-mRNA oder -cDNA amplifizieren kann, oder ein Mittel zum Nachweis von MGC4504-Protein verwendet, bei dem es sich um einen spezifischen MGC4504-Antikörper handelt, oder ein Mittel zum Nachweis von Mutationen in der MGC4504-Nukleinsäure verwendet, bei dem es sich um eine Nukleinsäuresonde oder einen Primersatz handelt, der MGC4504-Nukleinsäure amplifizieren kann, oder ein Mittel zum Nachweis von Mutationen in dem MGC4504-Protein verwendet, bei dem es sich um einen spezifischen Antikörper handelt, oder ein Mittel zum spezifischen Nachweis von genomischer MGC4504-DNA verwendet, das eine spezifische MGC4504-Nukleinsäuresonde oder ein(en) Primersatz, der genomische MGC4504-DNA oder MGC4504-cDNA amplifizieren kann, umfasst oder ist.

5. Verwendung nach Anspruch 4, wobei das Mittel ein Primer ist, der aus der Nukleotidsequenz gemäß SEQ ID NR: 8 besteht oder eine Nukleotidsequenz gemäß SEQ ID NR: 9 hat, oder eine Nukleinsäuresonde, die aus der Nukleotidsequenz gemäß SEQ ID NR: 10 besteht.

6. Verfahren zur Diagnose von metabolischem Syndrom, Fettleibigkeit, Dyslipidämie, Diabetes mellitus Typ I oder II oder Insulinresistenz und vorzugsweise Insulinresistenz oder einer Prädisposition für metabolisches Syndrom, Fettleibigkeit, Dyslipidämie, Diabetes mellitus Typ I oder II oder Insulinresistenz und vorzugsweise Insulinresistenz, bei dem man die Menge an MGC4504 analysiert, die in einer isolierten Probe von einem Individuum vorhanden ist, wobei eine verringerte MGC4504-Menge in der Probe im Vergleich zu einer oder mehreren Referenzproben die Prädisposition oder Erkrankung anzeigt und wobei die Referenzprobe eine isolierte Probe mit einer durchschnittlichen Aktivität oder Menge an MGC4504 und/oder eine Probe von einem Donor ist, der keine Erkrankung oder Prädisposition für eine Erkrankung hat, die mit einer Fehlfunktion des Kohlenhydrat- oder Lipidstoffwechsels in Zusammenhang steht oder davon verursacht wird.

7. Verfahren zur Diagnose von metabolischem Syndrom, Fettleibigkeit, Dyslipidämie, Diabetes mellitus Typ I oder II oder Insulinresistenz und vorzugsweise Insulinresistenz oder einer Prädisposition für metabolisches Syndrom, Fettleibigkeit, Dyslipidämie, Diabetes mellitus Typ I oder II oder Insulinresistenz und vorzugsweise Insulinresistenz, bei dem eine isolierte Probe von einem Individuum auf Mutationen von MGC4504 im Vergleich zu einer Referenzsequenz von MGC4504 analysiert, wobei das Vorliegen einer Mutation die Erkrankung oder die Prädisposition anzeigt.

8. Verfahren zur Diagnose von metabolischem Syndrom, Fettleibigkeit, Dyslipidämie, Diabetes mellitus Typ I oder II oder Insulinresistenz und vorzugsweise Insulinresistenz oder einer Prädisposition für metabolisches Syndrom, Fettleibigkeit, Dyslipidämie, Diabetes mellitus Typ I oder II oder Insulinresistenz und vorzugsweise Insulinresistenz, bei dem man eine isolierte Probe von einem Individuum auf eine gestörte oder verringerte MGC4504-Aktivität im Vergleich zu einer Referenzprobe analysiert, wobei das Vorliegen einer gestörten oder einer verringerten Aktivität die Erkrankung oder die Prädisposition anzeigt und wobei die Referenzprobe eine isolierte Probe mit einer durchschnittlichen Aktivität oder Menge an MGC4504 und/oder eine Probe von einem Donor ist, der keine Erkrankung oder Prädisposition für eine Erkrankung hat, die mit einer Fehlfunktion des Kohlenhydrat- oder Lipidstoffwechsels in Zusammenhang steht oder davon verursacht wird.

9. Kit zur Identifikation von metabolischem Syndrom, Fettleibigkeit, Dyslipidämie, Diabetes mellitus Typ I oder II oder Insulinresistenz und vorzugsweise Insulinresistenz oder einer Prädisposition für metabolisches Syndrom, Fettleibigkeit, Dyslipidämie, Diabetes mellitus Typ I oder II oder Insulinresistenz und vorzugsweise Insulinresistenz, das mindestens ein Mittel zum Nachweis von MGC4504 umfasst, wobei es sich bei dem Mittel um einen Primer, der aus der Nukleotidsequenz gemäß SEQ ID NR: 8 besteht oder eine Nukleotidsequenz gemäß SEQ ID NR: 9 hat, oder eine Nukleinsäuresonde handelt, die aus der Nukleotidsequenz gemäß SEQ ID NR: 10 besteht.

10. MGC4504 oder ein funktionelles Fragment oder Derivat davon oder eine Zusammensetzung von Substanzen, die MGC4504 oder ein funktionelles Fragment oder Derivat davon umfasst, für die Verwendung bei einem Verfahren zur Behandlung oder Vorbeugung von metabolischem Syndrom, Fettleibigkeit, Dyslipidämie, Diabetes mellitus Typ I oder II oder Insulinresistenz und vorzugsweise Insulinresistenz, wobei die Funktion des MGC4504-Proteinfragments definiert ist als die Fähigkeit, die Insulinfreisetzung aus den beta-Zellen des Pankreas zu verbessern/zu erhöhen, die Insulin-stimulierte Glucose-Lagerung zu verbessern/zu erhöhen, die Insulin-Signalgebung oder die Insulinwirkung zu beeinflussen oder die Insulin-stimulierte Glucose-Aufnahme zu erhöhen, und wobei das Derivat der MGC4504-Nukleinsäure eine Phosphorthioat-Modifikation umfasst und wobei es sich bei dem Derivat des MGC4504-Proteins oder dem Fragment davon um das Protein oder das Fragment davon, gekoppelt an ein zellpermeierendes Phosphopeptid, handelt.

11. In vitro-Verwendung einer Zelle, die MGC4504 heterolog exprimiert, zur Identifizierung von Substanzen, die zur Behandlung oder Vorbeugung von metabolischem Syndrom, Fettleibigkeit, Dyslipidämie, Diabetes mellitus Typ I oder II oder Insulinresistenz und vorzugsweise Insulinresistenz wirksam sind.

12. Verwendung eines nicht-menschlichen Tiers, das MGC4504 heterolog exprimiert, zur Identifizierung von Substanzen, die zur Behandlung oder Vorbeugung von metabolischem Syndrom, Fettleibigkeit, Dyslipidämie, Diabetes mellitus Typ I oder II oder Insulinresistenz und vorzugsweise Insulinresistenz wirksam sind.

13. In vitro-Verwendung einer MGC4504-Knockout-Zelle zur Identifizierung von Substanzen, die zur Behandlung oder Vorbeugung von metabolischem Syndrom, Fettleibigkeit, Dyslipidämie, Diabetes mellitus Typ I oder II oder Insulinresistenz und vorzugsweise Insulinresistenz wirksam sind.

14. Verwendung eines nicht-menschlichen MGC4504-Knockout-Tiers zur Identifizierung von Substanzen, die zur Behandlung oder Vorbeugung von metabolischem Syndrom, Fettleibigkeit, Dyslipidämie, Diabetes mellitus Typ I oder II oder Insulinresistenz und vorzugsweise Insulinresistenz wirksam sind.

15. Primer, der aus der Nukleotidsequenz gemäß SEQ ID NR: 8 besteht oder eine Nukleotidsequenz gemäß SEQ ID NR: 9 hat.

16. Nukleinsäuresonde, die aus der Nukleotidsequenz gemäß SEQ ID NR: 10 besteht.

17. Hochdurchsatz-Screening (HTS), das ein Verfahren nach Anspruch 2 oder 3 umfasst.

18. Verwendung nach Anspruch 1, MGC4504 oder ein funktionelles Fragment davon oder eine Zusammensetzung von Substanzen, die MGC4504 oder ein funktionelles Fragment davon umfasst, nach Anspruch 10, Verfahren nach Anspruch 2 oder HTS nach Anspruch 17, wobei MGC4504, das Derivat oder Fragment davon als isoliertes Molekül verwendet wird.

19. Verwendung nach Anspruch 1, Verfahren nach Anspruch 2 oder HTS nach Anspruch 17, wobei MGC4504, das Derivat oder Fragment in einem biochemischen oder zellulären Assay verwendet wird.

20. Verwendung, Verfahren oder HTS nach Anspruch 19, wobei der Assay ein zellulärer Assay ist und die Zellen transient oder stabil und vorzugsweise stabil derart transfiziert sind, dass sie MGC4504 heterolog exprimieren.

21. Verwendung, Verfahren oder HTS nach Anspruch. 20 oder Verwendung nach Anspruch 11 oder 12, wobei die Zelle eine Säugerzelle ist.

22. Verwendung, Verfahren oder HTS nach Anspruch 21, wobei die Zelle eine Nagetierzelle und vorzugsweise eine Mauszelle ist.

23. Verwendung, Verfahren oder HTS nach Anspruch 21, wobei die Zelle eine menschliche Zelle ist.

24. Verwendung nach Anspruch 12 oder 14, wobei das Tier ein Vertebrat oder ein Invertebrat, vorzugsweise ein Vertebrat, stärker bevorzugt ein nicht-menschlicher Säuger und noch stärker bevorzugt eine Maus oder eine Ratte ist.

25. Verwendung nach Anspruch 11 oder 13, wobei das heterolog exprimierte MGC4504 ein Transgen ist.

26. Verwendung nach Anspruch 1, MGC4504 oder ein funktionelles Fragment davon oder eine Zusammensetzung von Substanzen, die MGC4504 oder ein funktionelles Fragment davon umfasst, nach Anspruch 10, Verfahren nach Anspruch 2 oder 3 oder HTS nach Anspruch 17, wobei es sich bei MGC4504 um Säuger- und vorzugsweise menschliches MGC4504 handelt.

27. Verwendung nach Anspruch 4 oder Verfahren nach einem der Ansprüche 6 bis 8, wobei das Individuum ein menschliches Individuum ist.

28. Verwendung nach Anspruch 1, MGC4504 oder ein funktionelles Fragment davon oder eine Zusammensetzung von Substanzen, die MGC4504 oder ein funktionelles Fragment davon umfasst, nach Anspruch 10, Verfahren nach einem der Ansprüche 2, 6 oder 7, Kit nach Anspruch 9 oder HTS nach Anspruch 17, wobei MGC4504 oder das Derivat oder Fragment davon eine Nukleinsäure ist.

29. Verwendung, Verfahren oder Hochdurchsatz-Screening nach Anspruch 28 oder Verfahren nach Anspruch 3, wobei die Nukleinsäure eine der folgenden Sequenzen umfasst oder aus einer der folgenden Sequenzen besteht:
a. die Sequenz gemäß SEQ ID NR: 1 oder 14,
b. eine Sequenz, die mit einer Sequenz gemäß a) unter Bedingungen hoher Stringenz hybridisieren kann und für ein Protein oder Polypeptid mit MGC4504-Funktion kodiert,
c. eine Sequenz, die von einer Sequenz gemäß a) oder b) aufgrund der Degeneration des genetischen Codes herrührt und für ein Protein oder Polypeptid mit MGC4504-Funktion kodiert,
d. ein Fragment von einer der Sequenzen gemäß a), b) oder c), das für ein Polypeptid mit MGC4504-Funktion kodiert, wobei das Protein oder Polypeptid eine Polypeptidsequenz hat, die die Sequenz gemäß SEQ ID NR: 2 umfasst oder daraus besteht,
wobei die MGC4504-Funktion gemäß b. bis d. definiert ist als die Fähigkeit, die Insulinfreisetzung aus den beta-Zellen des Pankreas zu verbessern/zu erhöhen, die Insulin-stimulierte Glucose-Lagerung zu verbessern/zu erhöhen, die Insulin-Signalgebung oder die Insulinwirkung zu beeinflussen oder die Insulin-stimulierte Glucose-Aufnahme zu erhöhen.

30. Verwendung nach Anspruch 1, MGC4504 oder ein funktionelles Fragment davon oder eine Zusammensetzung von Substanzen, die MGC4504 oder ein funktionelles Fragment davon umfasst, nach Anspruch 10, Verfahren nach einem der Ansprüche 2, 6 oder 7, Kit nach Anspruch 9 oder Hochdurchsatz-Screening nach Anspruch 17, wobei MGC4504 oder das Derivat oder Fragment davon ein Protein oder Polypeptid ist.

31. Verwendung, Verfahren, Kit oder Hochdurchsatz-Screening nach Anspruch 30, Verwendung nach Anspruch 1 oder Verfahren nach Anspruch 2, wobei das Polypeptid oder Protein durch eine der folgenden Sequenzen kodiert wird:
a. die Sequenz gemäß SEQ ID NR: 1 oder 14,
b. eine Sequenz, die mit der Sequenz gemäß a) unter Bedingungen hoher Stringenz hybridisieren kann und für ein Protein oder Polypeptid mit MGC4504-Funktion kodiert,
c. eine Sequenz, die von einer Sequenz gemäß a) oder b) aufgrund der Degeneration des genetischen Codes herrührt und für ein Protein oder Polypeptid mit MGC4504-Funktion kodiert,
d. ein Fragment von einer der Sequenzen gemäß a), b) oder c), das für ein Protein oder Polypeptid mit MGC4504-Funktion kodiert,
wobei die MGC4504-Funktion gemäß b. bis d. definiert ist als die Fähigkeit, die Insulinfreisetzung aus den beta-Zellen des Pankreas zu verbessern/zu erhöhen, die Insulin-stimulierte Glucose-Lagerung zu verbessern/zu erhöhen, die Insulin-Signalgebung oder die Insulinwirkung zu beeinflussen oder die Insulin-stimulierte Glucose-Aufnahme zu erhöhen.

32. Verwendung, Verfahren, Kit, Hochdurchsatz-Screening nach Anspruch 30 oder 31, wobei das Protein oder Polypeptid die Sequenz gemäß SEQ ID NR: 2 umfasst oder daraus besteht.

33. Verwendung, Verfahren oder Hochdurchsatz-Screening nach einem der vorhergehenden Ansprüche, wobei die Modulation eine Aktivierung ist.

34. Verfahren nach Anspruch 3, wobei das Reportergen ein Gen ist, das Renilla- oder Leuchtkäfer-Luciferase, grün oder blau fluoreszierendes Protein, beta-Galactosidase oder Chloramphenicol-Acetyltransferase kodiert.

35. Verfahren oder Verwendung nach einem der Ansprüche 2, 3, 11 oder 12, wobei die Zelle eine primäre Zelle ist oder zu einer Zelllinie gehört.

36. Verfahren oder Verwendung nach Anspruch 35, wobei die Zelle eine HEK293-, RIN-5F-, CHO- oder NIH3T3-Zelle ist.

37. Verwendung nach Anspruch 11, wobei die Zelle aus einem nicht-menschlichen transgenen Tier isoliert worden ist.

38. Verwendung nach Anspruch 13, wobei die Zelle aus einem nicht-menschlichen Knockout-Tier isoliert worden ist.

39. Verfahren nach Anspruch 3, wobei die Transfektion eine transiente oder stabile Transfektion und vorzugsweise eine stabile Transfektion ist.

40. Verfahren zur Behandlung nach Anspruch 8 oder 27, bei dem man den MGC4504-Fließgleichgewicht-Spiegel endogen erhöht.

41. Verfahren nach Anspruch 6, bei dem man ein Mittel zur spezifischen Analyse des MGC4504-mRNA-Spiegels verwendet, das eine spezifische MGC4504-Nukleinsäuresonde oder ein Primersatz ist, der spezifisch MGC4504-cDNA oder ein Fragment davon amplifizieren kann, wobei die Funktion des von dem Fragment kodierten MCG-Proteins definiert ist als die Fähigkeit, die Insulinfreisetzung aus den beta-Zellen des Pankreas zu verbessern/zu erhöhen, die Insulin-stimulierte Glucose-Lagerung zu verbessern/zu erhöhen, die Insulin-Signalgebung oder die Insulinwirkung zu beeinflussen oder die Insulin-stimulierte Glucose-Aufnahme zu erhöhen.

42. Verfahren nach Anspruch 6, bei dem man ein Mittel zur spezifischen Analyse des MGC4504-Proteinspiegels verwendet, das ein spezifischer MGC4504-Antikörper ist.

43. Verfahren nach Anspruch 6, das ein Mittel zum Nachweis von MGC4504-mRNA verwendet, das eine spezifische mRNA-Sonde oder ein Primersatz ist, der in der Lage ist, MGC4504-mRNA oder -cDNA zu amplifizieren.

44. Verfahren nach Anspruch 6, das ein Mittel zum Nachweis von MGC4504-Protein verwendet, das ein spezifischer MGC4504-Antikörper ist.

45. Verfahren nach Anspruch 7, das ein Mittel zum Nachweis von Mutationen in der MGC4504-Nukleinsäure verwendet, das eine Nukleinsäuresonde oder ein Primersatz ist, der in der Lage ist, MGC4504-Nukleinsäure zu amplifizieren.

46. Verfahren nach Anspruch 7, das ein Mittel zum Nachweis von Mutationen im MGC4504-Protein verwendet, das ein spezifischer Antikörper ist.

47. Verwendung einer Knockout-Zelle oder eines Knockout-Tiers nach Anspruch 13 oder 14, wobei es sich bei dem Knockout um einen funktionellen oder genomischen Knockout handelt.

## Revendications

1. Utilisation de la protéine MGC4504, dérivé ou fragment fonctionnel de celle-ci, ou un acide nucléique codant pour ladite protéine, dérivé ou fragment de celui-ci, pour l'identification de substances actives dans la prévention ou le traitement du syndrome métabolique, de l'obésité, d'une dyslipidémie, du diabète sucré de type I ou II ou de l'insulinorésistance et de préférence l'insulinorésistance, où la fonction du fragment de protéine MGC4504 est définie comme étant la capacité d'amélioration/augmentation de libération d'insuline par les cellules bêta pancréatiques, l'amélioration/augmentation de l'élimination du glucose stimulée par l'insuline, l'influence de la signalisation de l'insuline ou l'action de l'insuline ou l'augmentation de l'absorption de glucose stimulée par l'insuline et où le dérivé de l'acide nucléique comprend une modification phosphorothioate et où le dérivé de la protéine ou un fragment de celle-ci est la protéine ou un fragment de celle-ci couplée à un phosphopeptide de perméation cellulaire.

2. Procédé pour identifier des substances actives dans la prévention ou le traitement du syndrome métabolique, de l'obésité, d'une dyslipidémie, du diabète sucré de type I ou II ou de l'insulinorésistance et de préférence l'insulinorésistance comprenant :
a. la mise en contact d'une cellule, qui a une activité réduite ou une quantité de MGC4504, avec une substance d'essai ;
b. la détermination si la substance est capable d'augmenter l'activité ou la quantité de MGC4504 présent dans la cellule.

3. Procédé pour identifier des substances actives dans la prévention ou le traitement du syndrome métabolique, de l'obésité, d'une dyslipidémie, du diabète sucré de type I ou II ou de l'insulinorésistance et de préférence l'insulinorésistance comprenant :
a. la production d'une cellule transfectée avec un vecteur d'acide nucléique comprenant le promoteur MGC4504 ou un fragment fonctionnel de celui-ci fonctionnellement couplé à un gène rapporteur ou un fragment fonctionnel de celui-ci ;
b. la production d'une cellule transfectée avec un vecteur témoin qui comprend un gène rapporteur ou un fragment fonctionnel de celui-ci n'étant pas fonctionnellement couplé à un promoteur MGC4504 fonctionnel ;
c. la détermination de l'activité de gène rapporteur de la cellule selon a) et b) en présence d'une substance d'essai ;
d. la détermination de l'activité du gène rapporteur en l'absence de ladite substance, dans lequel une substance active est une substance capable d'augmenter l'activité du gène rapporteur selon a) sans augmenter l'activité du gène rapporteur de b).

4. Utilisation d'un moyen pour la détection de MGC4504 pour diagnostiquer le syndrome métabolique, l'obésité, une dyslipidémie, le diabète sucré de type I ou II ou l'insulinorésistance et de préférence l'insulinorésistance ou une prédisposition au syndrome métabolique, à l'obésité, à une dyslipidémie, au diabète sucré de type I ou II ou à l'insulinorésistance et de préférence à l'insulinorésistance dans un échantillon isolé d'un individu, de manière à utiliser un moyen pour détecter l'ARNm de MGC4504 qui est une sonde d'ARNm ou un ensemble d'amorce capable d'amplifier l'ARNm ou l'ADNc de MGC4504, ou en utilisant un moyen pour détecter la protéine MGC4504 qui est un anticorps spécifique anti-MGC4504, ou en utilisant un moyen pour détecter des mutations dans l'acide nucléique MGC4504 qui est une sonde d'acide nucléique ou un ensemble d'amorce capable d'amplifier l'acide nucléique MGC4504, ou en utilisant un moyen pour détecter des mutations dans la protéine MGC4504 qui est un anticorps spécifique ou en utilisant un moyen pour détecter spécifiquement l'ADN génomique MGC4504 qui comprend ou est une sonde d'acide nucléique MGC4504 spécifique ou un ensemble d'amorce capable d'amplifier MGC4504 génomique ou ADNc ou un fragment de celui-ci.

5. Utilisation de la revendication 4, **caractérisée en ce que** le moyen est une amorce constituée de la séquence nucléotidique selon SEQ ID NO: 8 ou avec une séquence nucléotidique selon SEQ ID NO: 9, ou une sonde d'acide nucléique constituée de la séquence nucléotidique selon SEQ ID NO: 10.

6. Procédé de diagnostic du syndrome métabolique, de l'obésité, d'une dyslipidémie, du diabète sucré de type I ou II ou de l'insulinorésistance et de préférence l'insulinorésistance ou une prédisposition au syndrome métabolique, l'obésité, une dyslipidémie, le diabète sucré de type I ou II ou l'insulinorésistance et de préférence l'insulinorésistance comprenant l'analyse de la quantité de MGC4504 présent dans un échantillon isolé d'un individu, où une quantité abaissée de MGC4504 présent dans ledit échantillon par rapport à un ou plusieurs échantillons de référence est indicatrice de la prédisposition ou de la maladie, et où l'échantillon de référence est un échantillon isolé ayant une activité ou quantité de MGC4504 moyenne et/ou un échantillon d'un donneur n'ayant pas une maladie ou une prédisposition à une maladie associée à ou causée par un dysfonctionnement du métabolisme des glucides ou des lipides.

7. Procédé de diagnostic du syndrome métabolique, de l'obésité, d'une dyslipidémie, du diabète sucré de type I ou II ou de l'insulinorésistance et de préférence l'insulinorésistance ou une prédisposition au syndrome métabolique, à l'obésité, à une dyslipidémie, au diabète sucré de type I ou II ou à l'insulinorésistance et de préférence à l'insulinorésistance comprenant l'analyse d'un échantillon isolé pour des mutations de MGC4504 par rapport à une séquence de référence de MGC4504, où la présence d'une mutation est indicatrice de la maladie ou la prédisposition.

8. Procédé de diagnostic du syndrome métabolique, de l'obésité, d'une dyslipidémie, du diabète sucré de type I ou II ou de l'insulinorésistance et de préférence l'insulinorésistance ou une prédisposition au syndrome métabolique, à l'obésité, à une dyslipidémie, au diabète sucré de type I ou II ou à l'insulinorésistance et de préférence à l'insulinorésistance comprenant l'analyse d'un échantillon isolé pour une activité MGC4504 altérée ou abaissée par rapport à un échantillon de référence, où la présence d'une activité altérée ou abaissée est indicatrice de la maladie ou la prédisposition, et où l'échantillon de référence est un échantillon isolé ayant une activité ou quantité moyenne de MGC4504 et/ou un échantillon d'un donneur n'ayant pas une maladie ou une prédisposition à une maladie associée à ou causée par un dysfonctionnement du métabolisme des glucides ou des lipides.

9. Kit pour l'identification du syndrome métabolique, de l'obésité, d'une dyslipidémie, du diabète sucré de type I ou II ou de l'insulinorésistance et de préférence l'insulinorésistance ou une prédisposition au syndrome métabolique, à l'obésité, à une dyslipidémie, au diabète sucré de type I ou II ou à l'insulinorésistance et de préférence à l'insulinorésistance comprenant au moins un moyen pour la détection de MGC4504, où le moyen est une amorce constituée de la séquence nucléotidique selon SEQ ID-NO: 8 ou avec une séquence nucléotidique selon SEQ ID NO: 9 ou une sonde d'acide nucléique constituée de la séquence nucléotidique selon SEQ ID NO: 10.

10. MGC4504 ou fragment fonctionnel ou dérivé de celui-ci ou composition de matière comprenant MGC4504 ou un fragment fonctionnel ou dérivé de celui-ci pour utilisation dans un procédé pour le traitement ou la prévention du syndrome métabolique, de l'obésité, d'une dyslipidémie, du diabète sucré de type I ou II ou de l'insulinorésistance et de préférence l'insulinorésistance, où la fonction du fragment de protéine MGC4504 est définie comme étant la capacité d'amélioration / augmentation de la libération d'insuline par les cellules bêta pancréatiques, l'amélioration/augmentation de l'élimination du glucose stimulée par l'insuline, l'influence de la signalisation de l'insuline ou l'action de l'insuline ou l'augmentation de l'absorption de glucose stimulée par l'insuline et où le dérivé de l'acide nucléique MGC4504 comprend une modification phosphorothioate et où le dérivé de la protéine MGC4504 ou un fragment de celle-ci est la protéine ou un fragment de celle-ci couplée à un phosphopeptide de perméation cellulaire.

11. Utilisation in vitro d'une cellule exprimant de façon hétérologue MGC4504 pour l'identification de substances actives dans le traitement ou la prévention du syndrome métabolique, de l'obésité, d'une dyslipidémie, du diabète sucré de type I ou II ou de l'insulinorésistance et de préférence de l'insulinorésistance.

12. Utilisation d'un animal non humain exprimant de façon hétérologue MGC4504 pour l'identification de substances actives dans le traitement ou la prévention du syndrome métabolique, de l'obésité, d'une dyslipidémie, du diabète sucré de type I ou II ou de l'insulinorésistance et de préférence l'insulinorésistance.

13. Utilisation in vitro d'une cellule knockout pour MGC4504 pour l'identification de substances actives dans le traitement ou la prévention du syndrome métabolique, de l'obésité, d'une dyslipidémie, du diabète sucré de type I ou II ou de l'insulinorésistance et de préférence de l'insulinorésistance.

14. Utilisation d'un animal non humain knockout pour MGC4504 pour l'identification de substances actives dans le traitement ou la prévention du syndrome métabolique, de l'obésité, d'une dyslipidémie, du diabète sucré de type I ou II ou de l'insulinorésistance et de préférence de l'insulinorésistance.

15. Amorce constituée de la séquence nucléotidique selon SEQ ID NO: 8 ou avec une séquence nucléotidique selon SEQ ID NO: 9.

16. Sonde d'acide nucléique constituée de la séquence nucléotidique selon SEQ ID No: 10.

17. Criblage à rendement élevé (HTS) comprenant un procédé selon la revendication 2 ou 3.

18. Utilisation selon la revendication 1, MGC4504 ou un fragment fonctionnel de celui-ci ou composition de matière comprenant MGC4504 ou un fragment fonctionnel de celui-ci selon la revendication 10, procédé selon la revendication 2 ou HTS selon la revendication 17, où MGC4504, le dérivé ou fragment de celui-ci est utilisé sous la forme d'une molécule isolée.

19. Utilisation selon la revendication 1, procédé selon la revendication 2, ou HTS selon la revendication 17, où MGC4504, le dérivé ou fragment de celui-ci est utilisé dans un essai biochimique ou cellulaire.

20. Utilisation, procédé ou HTS selon la revendication 19, où l'essai est un essai cellulaire et les cellules sont transfectées temporairement ou de façon stable et de préférence transfectées de façon stable pour exprimer de façon hétérologue MGC4504.

21. Utilisation, procédé ou HTS selon la revendication 20 ou utilisation selon la revendication 11 ou 12, où la cellule est une cellule de mammifère.

22. Utilisation, procédé ou HTS selon la revendication 21, dans laquelle la cellule est une cellule de rongeur et de préférence une cellule murine.

23. Utilisation, procédé ou HTS selon la revendication 21, où la cellule est une cellule humaine.

24. Utilisation selon la revendication 12 ou 14, dans laquelle l'animal est un vertébré ou un invertébré, de préférence un vertébré, plus préférablement un mammifère non humain et encore plus préférablement une souris ou un rat.

25. Utilisation selon la revendication 11 ou 13, où MGC4504 exprimé de façon hétérologue est un transgène.

26. Utilisation selon la revendication 1, MGC4504 ou un fragment fonctionnel de celui-ci ou une composition de matière comprenant MGC4504 ou un fragment fonctionnel de celui-ci selon la revendication 10, procédé selon la revendication 2 ou 3 ou HTS selon la revendication 17, où MGC4504 est MGC4504 de mammifère et de préférence humaine.

27. Utilisation selon la revendication 4 ou procédé selon une des revendications 6 à 8, où l'individu est un individu humain.

28. Utilisation selon la revendication 1, MGC4504 ou un fragment fonctionnel de celui-ci ou composition de matière comprenant MGC4504 ou un fragment fonctionnel de celui-ci selon la revendication 10, procédé selon une des revendications 2, 6 ou 7, kit selon la revendication 9, ou HTS selon la revendication 17, où MGC4504 ou le dérivé ou fragment de celui-ci est un acide nucléique.

29. Utilisation, procédé ou criblage à rendement élevé selon la revendication 28 ou procédé selon la revendication 3, où l'acide nucléique comprend ou est constitué d'une des séquences suivantes :
a. la séquence selon SEQ ID NO: 1 ou 14 ;
b. une séquence capable de s'hybrider avec une séquence selon a) dans des conditions de stringence élevée et codant pour une protéine ou un polypeptide ayant une fonction MGC4504 ;
c. une séquence dérivée d'une séquence selon a) ou b) en raison de la dégénérescence du code génétique et codant pour une protéine ou un polypeptide ayant la fonction MGC4504 ;
d. un fragment d'une des séquences selon a), b) ou c), codant pour un polypeptide ayant une fonction MGC4504, où la protéine ou polypeptide a une séquence polypeptidique comprenant ou constituée de la séquence selon SEQ ID NO: 2 ;
où la fonction MGC4504 selon b. à d. est définie comme étant la capacité d'amélioration / augmentation de la libération d'insuline par les cellules bêta pancréatiques, l'amélioration/augmentation de l'élimination du glucose stimulée par l'insuline, l'influence de la signalisation de l'insuline ou l'action de l'insuline ou l'augmentation de l'absorption-de glucose stimulée par l'insuline.

30. Utilisation selon la revendication 1, MGC4504 ou un fragment fonctionnel de celui-ci ou une composition de matière comprenant MGC4504 ou un fragment fonctionnel de celui-ci selon la revendication 10, procédé selon une des revendications 2, 6, ou 7, kit selon la revendication 9 ou criblage à rendement élevé selon la revendication 17, où MGC4504 ou le dérivé ou fragment de celui-ci est une protéine ou un polypeptide.

31. Utilisation, procédé, kit ou criblage à rendement élevé selon la revendication 30, utilisation selon la revendication 1 ou procédé selon la revendication 2, où le polypeptide ou la protéine est codé par une des séquences suivantes :
a. la séquence selon SEQ ID NO: 1 ou 14 ;
b. une séquence capable de s'hybrider avec la séquence selon a) dans dès conditions de stringence élevée et codant pour une protéine ou un polypeptide ayant une fonction MGC4504 ;
c. une séquence dérivée d'une séquence selon a) ou b) en raison de la dégénérescence du code génétique et codant pour une protéine ou un polypeptide ayant la fonction MGC4504 ;
d. un fragment d'une des séquences selon a), b) ou c), codant pour une protéine ou un polypeptide ayant une fonction MGC4504 ;
où la fonction MGC4504 selon b. à d. est définie comme étant la capacité d'amélioration / augmentation de la libération d'insuline par les cellules bêta pancréatiques, l'amélioration / augmentation de l'élimination du glucose stimulée par l'insuline, l'influence de la signalisation de l'insuline ou l'action de l'insuline ou l'augmentation de l'absorption de glucose stimulée par l'insuline.

32. Utilisation, procédé, kit, criblage à rendement élevé selon la revendication 30 ou 31, où la protéine ou le polypeptide comprend ou est constitué de la séquence selon SEQ ID NO: 2.

33. Utilisation, procédé ou criblage à rendement élevé selon une des revendications précédentes où la modulation est une activation.

34. Procédé selon la revendication 3, dans lequel le gène rapporteur est un gène codant pour la luciférase de Renilla ou de luciole, la protéine fluorescente verte ou bleue, la bêta-galactosidase, ou la chloramphénicol-acétyl-transférase.

35. Procédé ou utilisation selon une des revendications 2, 3, 11 ou 12, où la cellule est une cellule primaire ou appartient à une lignée cellulaire.

36. Procédé ou utilisation selon la revendication 35, où la cellule est une cellule HEK293, RIN-5F, CHO ou NTH3T3.

37. Utilisation selon la revendication 11, où la cellule est isolée à partir d'un animal transgénique non humain.

38. Utilisation selon la revendication 13, où la cellule est isolée à partir d'un animal knockout non humain.

39. Procédé selon la revendication 3, dans lequel la transfection est une transfection transitoire et stable et de préférence une transfection stable.

40. Procédé de traitement selon la revendication 8 ou 27 comprenant l'augmentation endogène du taux stationnaire de MGC4504.

41. Procédé selon la revendication 6 comprenant l'utilisation d'un moyen pour analyser spécifiquement le taux d'ARNm MGC4504 qui est une sonde d'acide nucléique MGC4504 spécifique ou un ensemble d'amorce capable d'amplifier spécifiquement l'ADNc de MGC4504 ou un fragment de celui-ci, où la fonction de la protéine MCG codée par le fragment est définie comme étant la capacité d'augmentation/amélioration de la libération d'insuline par les cellules bêta pancréatiques, amélioration / augmentation de l'élimination du glucose stimulée par l'insuline, influence de la signalisation de l'insuline ou l'action de l'insuline ou augmentation de l'absorption de glucose stimulée par l'insuline.

42. Procédé selon la revendication 6 comprenant l'utilisation d'un moyen pour analyser spécifiquement le taux de protéine MGC4504 qui est un anticorps anti-MGC4504 spécifique.

43. Procédé selon la revendication 6 utilisant un moyen pour détecter l'ARNm de MGC4504 qui est une sonde d'ARNm ou un ensemble d'amorce capable d'amplifier l'ARNm ou l'ADNc de MGC4504.

44. Procédé selon la revendication 6 utilisant des moyens pour détecter la protéine MGC4504 qui est un anticorps anti-MGC4504 spécifique.

45. Procédé selon la revendication 7 utilisant un moyen pour détecter des mutations dans l'acide nucléique MGC4504 qui est une sonde d'acide nucléique ou un ensemble d'amorce capable d'amplifier l'acide nucléique MGC4504.

46. Procédé selon la revendication 7 utilisant un moyen pour détecter des mutations dans la protéine MGC4504 qui est un anticorps spécifique.

47. Utilisation d'une cellule knockout ou animal selon la revendication 13 ou 14, où le knockout est un knockout (inactivation) fonctionnel ou génomique.
